# EUROPEAN PATENT APPLICATION

(11) **EP 3 135 297 A1**
(43) Date of publication of application: **01.03.2017**
(21) Application number: 15772775.1
(22) Date of filing: 03.04.2015
(51) Int. Cl.: A61K 45/00, A61K 45/06, A61K 47/24, A61P 9/10, A61P 13/12, A61P 17/00, A61P 17/04, A61P 17/06, A61P 19/02, A61P 27/02, A61P 29/00, A61P 35/00, A61P 37/08, A61P 43/00

(54) **DRUG DELIVERY PROMOTER CONTAINING SUBSTANCE FOR ACTIVATING LYSOPHOSPHOLIPID RECEPTORS**

(30) Priority: 04.04.2014 JP 2014077622; 01.09.2014 JP 2014177272
(71) Applicant: Osaka University, Suita-shi, Osaka 565-0871 (JP)
(72) Inventor: TAKAKURA, Nobuyuki, Suita-shi Osaka 565-0871 (JP); NAITO, Hisamichi, Suita-shi Osaka 565-0871 (JP); TAKARA, Kazuhiro, Suita-shi Osaka 565-0871 (JP)
(74) Representative: Witte, Weller & Partner Patentanwälte mbB
(86) International application number: PCT/JP2015/060666
(87) International publication number: WO 2015/152412

(57) **Abstract**

The present invention provides a drug delivery enhancer comprising, as an active ingredient, a substance that activates a lysophospholipid receptor, the enhancer being intended to be used for enhancing the delivery of a therapeutic drug for a disease involving abnormal blood vessel formation to an affected area; a pharmaceutical composition for treating a disease involving abnormal blood vessel formation, the composition comprising, as an active ingredient, a substance that activates a lysophospholipid receptor; and a method for screening for a therapeutic drug for a disease involving abnormal blood vessel formation, the method comprising selecting a substance that activates a lysophospholipid receptor specifically expressed in vascular endothelial cells of an affected area.

SEQUENCE LISTING

<110>  OSAKA UNIVERSITY

<120>  AGENT FOR INCREASING EFFICIENCY OF DRUG DELIVERY COMPRISING
LYSOPHOSPHOLIPID RECEPTOR AGONIST

<130>  O11F5208

<150>  JP 2014-077622
<151>  2014-04-04

<150>  JP 2014-177272
<151>  2014-09-01

<160>  14

<170>  PatentIn version 3.1

<210>  1
<211>  20
<212>  DNA
<213>  Artificial Sequence

<220>
<223>  Primer

<400>  1
ccgcttccat ttccctattt                                                    20


<210>  2
<211>  20
<212>  DNA
<213>  Artificial Sequence

<220>
<223>  Primer

<400>  2
aaaaccgtga tgtgcctctc                                                    20


<210>  3
<211>  19
<212>  DNA
<213>  Artificial Sequence

<220>
<223>  Primer

<400>  3
ccatcaaagg ctggttcct                                                     19


<210>  4
<211>  20
<212>  DNA
<213>  Artificial Sequence

<220>
<223>  Primer

<400>  4
tccaagtcac agaggcagtg                                                   20


<210>  5
<211>  24
<212>  DNA
<213>  Artificial Sequence

<220>
<223>  Primer

<400>  5
ttccactttc ccttctacta cctg                                              24


<210>  6
<211>  20
<212>  DNA
<213>  Artificial Sequence

<220>
<223>  Primer

<400>  6
tccacagcaa taaccagcaa                                                   20


<210>  7
<211>  20
<212>  DNA
<213>  Artificial Sequence

<220>
<223>  Primer

<400>  7
gccctctctg atttgctttt                                                   20


<210>  8
<211>  20
<212>  DNA
<213>  Artificial Sequence

<220>
<223>  Primer

<400>  8
tcctcctggt cctgatggta                                                   20


<210>  9
<211>  19
<212>  DNA
<213>  Artificial Sequence

<220>
<223>  Primer

<400>  9
agcgatgaac tgtggaagg                                                    19

<210>  10
<211>  21
<212>  DNA
<213>  Artificial Sequence

<220>
<223>  Primer

<400>  10
gcaggaagat gatgagattg g                                                21


<210>  11
<211>  20
<212>  DNA
<213>  Artificial Sequence

<220>
<223>  Primer

<400>  11
tgtgccctac aacatcaacc                                                  20


<210>  12
<211>  21
<212>  DNA
<213>  Artificial Sequence

<220>
<223>  Primer

<400>  12
tcacttcttc taaccgacca g                                                21


<210>  13
<211>  20
<212>  DNA
<213>  Artificial Sequence

<220>
<223>  Primer

<400>  13
aactttggca ttgtggaagg                                                  20


<210>  14
<211>  20
<212>  DNA
<213>  Artificial Sequence

<220>
<223>  Primer

<400>  14
ggatgcaggg atgatgttct                                                  20

SEQUENCE LISTING

<110>  OSAKA UNIVERSITY

<120>  AGENT FOR INCREASING EFFICIENCY OF DRUG DELIVERY COMPRISING
LYSOPHOSPHOLIPID RECEPTOR AGONIST

<130>  O11F5208

<150>  JP 2014-077622
<151>  2014-04-04

<150>  JP 2014-177272
<151>  2014-09-01

<160>  14

<170>  PatentIn version 3.1

<210>  1
<211>  20
<212>  DNA
<213>  Artificial Sequence

<220>
<223>  Primer

<400>  1
ccgcttccat ttccctattt                                                    20


<210>  2
<211>  20
<212>  DNA
<213>  Artificial Sequence

<220>
<223>  Primer

<400>  2
aaaaccgtga tgtgcctctc                                                    20


<210>  3
<211>  19
<212>  DNA
<213>  Artificial Sequence

<220>
<223>  Primer

<400>  3
ccatcaaagg ctggttcct                                                     19


<210>  4
<211>  20
<212>  DNA
<213>  Artificial Sequence

<220>
<223>  Primer

<400>  4
tccaagtcac agaggcagtg                                                   20


<210>  5
<211>  24
<212>  DNA
<213>  Artificial Sequence

<220>
<223>  Primer

<400>  5
ttccactttc ccttctacta cctg                                              24


<210>  6
<211>  20
<212>  DNA
<213>  Artificial Sequence

<220>
<223>  Primer

<400>  6
tccacagcaa taaccagcaa                                                   20


<210>  7
<211>  20
<212>  DNA
<213>  Artificial Sequence

<220>
<223>  Primer

<400>  7
gccctctctg atttgctttt                                                   20


<210>  8
<211>  20
<212>  DNA
<213>  Artificial Sequence

<220>
<223>  Primer

<400>  8
tcctcctggt cctgatggta                                                   20


<210>  9
<211>  19
<212>  DNA
<213>  Artificial Sequence

<220>
<223>  Primer

<400>  9
agcgatgaac tgtggaagg                                                    19

<210>  10
<211>  21
<212>  DNA
<213>  Artificial Sequence

<220>
<223>  Primer

<400>  10
gcaggaagat gatgagattg g                                                21


<210>  11
<211>  20
<212>  DNA
<213>  Artificial Sequence

<220>
<223>  Primer

<400>  11
tgtgccctac aacatcaacc                                                  20


<210>  12
<211>  21
<212>  DNA
<213>  Artificial Sequence

<220>
<223>  Primer

<400>  12
tcacttcttc taaccgacca g                                                21


<210>  13
<211>  20
<212>  DNA
<213>  Artificial Sequence

<220>
<223>  Primer

<400>  13
aactttggca ttgtggaagg                                                  20


<210>  14
<211>  20
<212>  DNA
<213>  Artificial Sequence

<220>
<223>  Primer

<400>  14
ggatgcaggg atgatgttct                                                  20

## Description

### TECHNICAL FIELD

The present invention relates to a drug delivery enhancer comprising a substance that activates a lysophospholipid receptor, and a pharmaceutical composition comprising a substance that activates a lysophospholipid receptor.

### BACKGROUND ART

Blood vessel formation is involved in various diseases including tumors, diabetic retinopathy, age-related macular degeneration, chronic inflammatory diseases such as rheumatoid arthritis, acute inflammatory diseases such as infectious diseases, vascular malformations, and arteriosclerosis. Such diseases whose pathogenesis involves blood vessel formation are collectively called vascular diseases. De novo formation of blood vessels that occurs during the embryo development is called vasculogenesis. In contrast, new blood vessel formation from pre-existing vessels is called angiogenesis. Angiogenesis is involved in the blood vessel formation in various pathologies.

VEGF (vascular endothelial growth factor), known as a factor that stimulates the growth of vascular endothelium, plays a central role in all types of blood vessel formation, including physiological blood vessel formation that is observed in the period from the fetal stage to adolescence as well as pathological angiogenesis in the development of diseases. VEGF has a potent effect of inducing the growth of vascular endothelial cells. To initiate blood vessel formation, VEGF induces Src-mediated phosphorylation of the adherens junction protein VE-cadherin, resulting in the internalization of VE-cadherin in vascular endothelial cells, thereby loosening the junctions between vascular endothelial cells.

A normal vessel lumen is structurally stable due to adhesion of mural cells to vascular endothelial cells. Vascular endothelial cells tightly adhere to each other via various adhesion molecules, including VE-cadherin as described above, as well as claudin 5, integrins, and connexins, whereby the endothelial cells are controlled so as not to allow leakage of substances or cells from the blood vessels. Vascular endothelial cells also form desmosome together with mural cells. The desmosome controls the vascular permeability via molecular transport between vascular endothelial cells and vascular mural cells. Healthy blood vessels run parallel side by side.

Blood vessels in tumors have various abnormalities, including increased permeability, tortuous course, dilatation, saccular formation in some cases, and irregular branching patterns. Vascular endothelial cells in tumors also show abnormal morphology. Vascular mural cells covering endothelial cells are highly interspersed in the center of a tumor, and have low adhesion to vascular endothelial cells. Vessels covered by mural cells cannot be observed in many tumor regions. These abnormalities are mainly caused by over-secretion of VEGF in tumors.

Angiogenic inhibitors are often designed to target VEGF or its cognate receptors. Some angiogenic inhibitors have been successfully clinically applied, including neutralizing antibodies against VEGF, soluble VEGF receptors, and phosphorylation inhibitors against the VEGF receptors. In the early stage of their development, angiogenic inhibitors were expected to inhibit vascular formation in tumors and to stop the feeding of oxygen and nutrients, thereby inhibiting the growth of tumors. This approach is based on the concept of angiogenesis in tumors proposed by Dr. J. Folkman in the 1970s (Non-patent literature 1). In preclinical studies, monotherapy with an angiogenic inhibitor alone showed a significant anti-tumor effect in mice, but showed only a limited anti-tumor effect in humans. Combination therapy using an angiogenic inhibitor and an anticancer drug showed a higher tumor growth inhibitory effect than monotherapy with the anticancer drug alone (Non-patent literature 2).

Based on the above evidence, a new concept was proposed by Dr. R. Jain that the improved effect of the combination therapy of an angiogenic inhibitor and an anticancer drug is attributed to the normalization of the blood vessels in a tumor by the angiogenic inhibitor (Non-patent literature 3). That is, blockage of the intracellular signals of the VEGF receptors induced by VEGF inhibits the loosening of the junctions between vascular endothelial cells and normalizes the VEGF-mediated over-enhanced vascular permeability. Then, the pressure in the blood vessels that has been in equilibrium with the internal pressure in the tumor tissue can become higher than the internal pressure in the tumor tissue. As a result, the penetration of the anticancer drug from the blood vessels to the tumor is improved, and the anticancer drug exhibits a significantly improved effect.

After this discovery, the recovery of the vascular permeability of tumors for induction of efficient drug delivery to the tumors is considered to be a potentially effective approach to cancer therapy. Despite the above benefits, angiogenic inhibitors inhibit the survival of vascular endothelial cells, and induce cell death of vascular endothelial cells and their interacting vascular mural cells, thereby enhancing the ischemic environment of tumors. Hypoxia in tumors may cause malignant conversion of cancer cells that facilitates invasion and metastasis. Angiogenic inhibitors have been also reported to damage the blood vessels of normal tissues and cause severe adverse effects, such as hypertension, lung hemorrhage and renal dysfunction. Under the above circumstances, there has been a demand for the development of a drug that normalizes the vascular permeability of tumors without causing the regression of tumor blood vessels and without affecting normal blood vessels. In terms of diseases involving blood vessel abnormalities other than tumors, there has also been a demand for the development of a drug that normalizes blood vessels in pathological areas without affecting normal blood vessels.

### CITATION LIST

### NON-PATENT LITERATURE

Non-patent literature 1: Folkman J, et al: Isolation of a tumor factor responsible for angiogenesis. J Exp Med 133: 275-288, 1971.
Non-patent literature 2: Gerber HP, Ferrara N. Pharmacology and pharmacodynamics of bevacizumab as monotherapy or in combination with cytotoxic therapy in preclinical studies. Cancer Res 65; 671-680, 2005.
Non-patent literature 3: Jain RK: Normalization of tumor vasculature: An emerging concept in antiangiogenic therapy. Science 307: 58-62, 2005.

### SUMMARY OF INVENTION

### TECHNICAL PROBLEM

An object of the present invention is to find a substance that normalizes the permeability of blood vessels in an affected area without affecting normal blood vessels in a disease involving abnormal blood vessel formation, such as solid cancers, and to provide a novel application of such a substance.

### SOLUTION TO PROBLEM

The present invention has been made to solve the above problems and includes the following.
[1] A drug delivery enhancer comprising, as an active ingredient, a substance that activates a lysophospholipid receptor, the enhancer being intended to be used in combination with a therapeutic drug for a disease involving abnormal blood vessel formation, thereby enhancing the delivery of the therapeutic drug for a disease involving abnormal blood vessel formation to an affected area.
[2] The drug delivery enhancer of the above [1], wherein the substance that activates a lysophospholipid receptor is a lysophospholipid, a precursor thereof or a derivative thereof.
[3] The drug delivery enhancer of the above [1] or [2], wherein the disease involving abnormal blood vessel formation is a solid cancer, age-related macular degeneration, rheumatoid arthritis, psoriasis, scleroderma, systemic lupus erythematosus, vasculitis syndrome, vasculo-Behcet's disease, diabetic retinopathy, diabetic nephropathy, arteriosclerosis, chronic arteriosclerosis obliterans, Buerger's disease, pulmonary fibrosis, cerebral infarction, a serious infection or cardiac failure.
[4] The drug delivery enhancer of the above [3], wherein the disease involving abnormal blood vessel formation is a solid cancer.
[5] The drug delivery enhancer of any one of the above [1] to [4], wherein the lysophospholipid is one or more selected from lysophosphatidic acid, lysophosphatidylserine, lysophosphatidylcholine, lysophosphatidylethanolamine, lysophosphatidylinositol, lysophosphatidylglycerol, sphingosine-1-phosphate, sphingosylphosphorylcholine and platelet activating factor (PAF).
[6] The drug delivery enhancer of the above [5], wherein the lysophospholipid is lysophosphatidic acid.
[7] A pharmaceutical composition for treating a disease involving abnormal blood vessel formation, the composition comprising, as an active ingredient, a substance that activates a lysophospholipid receptor.
[8] The pharmaceutical composition of the above [7], wherein the substance that activates a lysophospholipid receptor is a lysophospholipid, a precursor thereof or a derivative thereof.
[9] The pharmaceutical composition of the above [8], which has one or more effects selected from inhibitory effect on tumor growth, inhibitory effect on cancer metastasis, enhancing effect on immunity against a tumor, and inhibitory effect on an increase in vascular permeability.
[10] The pharmaceutical composition of the above [8] or [9], which is used in combination with another drug for treating a disease involving abnormal blood vessel formation.
[11] The pharmaceutical composition of the above [10], wherein said another drug for treating a disease involving abnormal blood vessel formation is a therapeutic drug for a cancer.
[12] The pharmaceutical composition of the above [10] or [11], which enhances the delivery of said another drug used in combination to an affected area of a disease involving abnormal blood vessel formation.
[13] The pharmaceutical composition of any one of the above [7] to [12], wherein the disease involving abnormal blood vessel formation is a solid cancer, age-related macular degeneration, rheumatoid arthritis, psoriasis, scleroderma, systemic lupus erythematosus, vasculitis syndrome, vasculo-Behcet's disease, diabetic retinopathy, diabetic nephropathy, arteriosclerosis, chronic arteriosclerosis obliterans, Buerger's disease, pulmonary fibrosis, cerebral infarction, a serious infection or cardiac failure.
[14] The pharmaceutical composition of the above [13], wherein the disease involving abnormal blood vessel formation is a solid cancer.
[15] The pharmaceutical composition of any one of the above [7] to [14], wherein the lysophospholipid is one or more selected from lysophosphatidic acid, lysophosphatidylserine, lysophosphatidylcholine, lysophosphatidylethanolamine, lysophosphatidylinositol, lysophosphatidylglycerol, sphingosine-1-phosphate, sphingosylphosphorylcholine and platelet activating factor (PAF).
[16] The pharmaceutical composition of the above [15], wherein the lysophospholipid is lysophosphatidic acid.
[17] A method for screening for a therapeutic drug for a disease involving abnormal blood vessel formation, the method comprising selecting a substance that activates a lysophospholipid receptor specifically expressed in vascular endothelial cells of an affected area.

### ADVANTAGEOUS EFFECTS OF INVENTION

The drug delivery enhancer of the present invention comprising, as an active ingredient, a substance that activates a lysophospholipid receptor normalizes the vascular permeability in the affected area of a disease involving abnormal blood vessel formation without affecting normal blood vessels. Due to this effect, the drug delivery enhancer enhances the delivery of a drug used in combination to the affected area of a disease involving abnormal blood vessel formation, thereby allowing such a therapeutic drug used in combination to exert a high therapeutic effect even in a small amount. The pharmaceutical composition of the present invention comprising a substance that activates a lysophospholipid receptor as active ingredient can be used alone for the treatment of a disease involving abnormal blood vessel formation. In particular, use of the pharmaceutical composition of the present invention for the treatment of a solid cancer inhibits tumor growth and cancer cell metastasis and enhance immunity against tumors without inducing malignant conversion of cancer cells.

### BRIEF DESCRIPTION OF DRAWINGS

Figs. 1A and 1B show the structural changes in tumor blood vessels after administration of lysophosphatidic acid (LPA) to mice bearing Lewis lung cancer (LLC) cell tumors. Fig. 1A shows the confocal laser microscopic images of tumor tissue specimens containing vascular endothelial cells immunostained with anti-CD31 antibody. Fig. 1B is a chart of the average length of blood vessels in each group quantified with the vascular structure analysis software AngioTool.
Fig. 2 shows the structural changes in tumor blood vessels. The photographs are the confocal laser microscopic images of tumor tissue specimens containing vascular endothelial cells immunostained with anti-CD31 antibody after administration of sphingosine-1-phosphate (S1P) to LLC cell tumor-bearing mice.
Fig. 3 shows the scanning electron microscopic images showing the structural changes in the lumen of tumor blood vessels after administration of lysophosphatidic acid (LPA) to LLC cell tumor-bearing mice.
Fig. 4 shows the confocal laser microscopic images showing drug delivery from tumor blood vessels to tumor tissues after administration of lysophosphatidic acid (LPA) and doxorubicin to LLC cell tumor-bearing mice.
Fig. 5 shows the confocal laser microscopic images showing drug delivery from tumor blood vessels to tumor tissues after administration of lysophosphatidic acid (LPA) and FITC-labeled dextrans with different molecular weights (70 kDa or 2000 kDa) to LLC cell tumor-bearing mice.
Figs. 6A to 6C show the effect of the combined administration of lysophosphatidic acid (LPA) and 5-FU on LLC cell tumor-bearing mice. Fig. 6A shows the changes in tumor volume with time in each group. Fig. 6B is a photograph of the appearance of a representative mouse from each group. Fig. 6C shows the tumors harvested from a representative mouse from each group.
Figs. 7A and 7B show the effect of the combined administration of lysophosphatidic acid (LPA) and 5-FU in B16-BL6 cell tumor-bearing mice. Fig. 7A shows the changes in tumor volume with time in each group. Fig. 7B is a photograph of the appearance of a representative mouse from each group.
Fig. 8 shows the effect of the combined administration of lysophosphatidic acid (LPA) and 5-FU in colon 26 cell tumor-bearing mice. The chart shows the changes in tumor volume in each group.
Fig. 9 shows the effect of the combined administration of lysophosphatidic acid (LPA) and oxaliplatin in colon 26 cell tumor-bearing mice. The chart shows the changes in tumor volume with time in each group.
Figs. 10A and 10B show the expression of VE-cadherin in vascular endothelial cells in tumors after administration of lysophosphatidic acid (LPA) to LLC cell tumor-bearing mice. Fig. 10A shows the confocal laser microscopic images of tumor tissue specimens containing vascular endothelial cells immunostained with anti-VE-cadherin antibody. Fig. 10B is a chart showing the expression level (fluorescence intensity) of VE-cadherin determined from Z-stack images acquired along the direction indicated by the inclined white bar in Fig. 10A.
Fig. 11 shows the strength of the endothelial cell-cell adhesion (barrier function) in endothelial cells cultured in the presence of LPA or the LPA derivative VPC 31144 (S).
Fig. 12 shows the number of lung metastatic colonies derived from a primary lesion, counted after administration of lysophosphatidic acid (LPA) to B16-BL6 cell tumor-bearing mice.
Fig. 13 shows the real-time PCR analysis results of the expression levels of LPA receptors (LPARs) 1-6 in vascular endothelial cells of tumor tissues harvested from LLC cell tumor-bearing mice.
Fig. 14 shows the real-time PCR analysis results of the expression levels of LPA receptors (LPARs) 1-6 in MS-1 cells (mouse pancreatic vascular endothelial cell line).
Fig. 15 shows the comparison of the confocal laser microscopic images of LPAR4 knockdown MS-1 cells and control MS-1 cells cultured until confluence followed by immunofluorescent staining with anti-VE-cadherin antibody.
Fig. 16 shows the effect of the administration of lysophosphatidic acid (LPA) on edema caused by increased permeability of newly formed blood vessels in a murine model of hindlimb ischemia.
Fig. 17 shows the effect of the administration of lysophosphatidic acid (LPA) for inhibiting an increase in the vascular permeability of newly formed blood vessels in a murine model of age-related macular degeneration. The upper panels show the immunostaining of vascular endothelial cells with anti-CD31 antibody. The lower panels show the leakage of FITC-labeled dextran from newly formed blood vessels.

### DESCRIPTION OF EMBODIMENTS

A lysophospholipid is a phospholipid having one acyl group. Lysophospholipids are classified into two classes: one with a glycerol backbone and the other with a sphingosine backbone. Lysophospholipids include a large number of molecular species defined by a combination of a polar group and an acyl group bound to the backbone. Lysophospholipids are known as a lipid mediator that exhibits various biological activities by binding to a specific receptor. However, little was known about the physiological functions of lysophospholipids in a living body. In particular, nothing was known about their effects on the blood vessels in diseases involving abnormal blood vessel formation.

The inventors administered a member of the lysophospholipid family, lysophosphatidic acid (LPA), to tumor-bearing mice generated by subcutaneous inoculation of cancer cells. The inventors found that tumor blood vessels with tortuous course and irregular branching before administration resulted in fine vascular network similar to that observed in normal tissues. The inventors also found that the irregular luminal structure of the tumor blood vessels before LPA administration resulted in a smooth structure after LPA administration. The inventors further found that the excessively increased vascular permeability of the tumor blood vessels was reduced to the normal level by LPA administration. That is, the inventors found that LPA exhibits the following effects in diseases involving abnormal blood vessel formation such as a solid cancer: the effect of inducing the formation of a fine network of blood vessels to normalize the blood vessels, the effect of inducing the formation of a smooth vascular lumen, and the effect of normalizing the vascular permeability. The inventors also conducted further experiments using a murine model of hindlimb ischemia as a pathological model of Buerger's disease and chronic arteriosclerosis obliterans, and using a murine model of age-related macular degeneration. The inventors, as a result, found that LPA also exhibits the effect of normalizing the increased vascular permeability of newly formed blood vessels in tissues other than tumors, thereby ameliorating the pathologies. These findings indicated that LPA has the effect of normalizing abnormal blood vessels in diseases involving abnormal blood vessel formation. Based on this, therefore, a lysophospholipid was expected to be useful as an active ingredient of a drug for normalizing blood vessels in diseases involving abnormal blood vessel formation.

Six subtypes of the LPA receptor (LPAR) have been discovered (LPARsl-6) thus far. LPARsl-3 are highly expressed in cancer cells, and in in vitro culture, the growth of cancer cells is induced by LPA. The inventors analyzed the LPARs expressed in vascular endothelial cells using tumor-bearing mice, and found that LPAR1, LPAR4 and LPAR6 were expressed. Vascular endothelial cells with LPAR4 knockdown showed disrupted cell-cell adhesion. The results revealed that at least LPAR4 is involved in the normalization of tumor blood vessels.

It may be concluded, based on the overall results, that a cancer can be effectively treated without stimulating the growth and mobility of cancer cells by specific activation of LPARs that are specifically expressed in vascular endothelial cells in tumors without the activation of LPARsl-3, which are highly expressed in cancer cells. In other words, an LPA receptor agonist that specifically regulates LPAR4 or LPAR6 may be useful as an active ingredient of a drug for normalizing blood vessels, as in the case of a lysophospholipid. In addition, an agonist that regulates an LPA receptor, including currently identified LPARsl-6 and as yet unidentified LPA receptors, and thereby induces the normalization of blood vessels may be useful as an active ingredient of a drug for normalizing blood vessels.

### Drug delivery enhancer

The present invention provides a drug delivery enhancer for enhancing the delivery of a therapeutic drug for a disease involving abnormal blood vessel formation to an affected area. The drug delivery enhancer of the present invention comprises, as an active ingredient, a substance that activates a lysophospholipid receptor and is intended to be used in combination with a therapeutic drug for a disease involving abnormal blood vessel formation. Abnormal blood vessel formation leads to a reduction in the blood flow in the affected area and an excessive increase in the vascular permeability. In a solid cancer, for example, abnormal blood vessel formation causes an increase in the fluid pressure in the tumor stroma, leading to no difference in osmotic pressure between the tumor stroma and the blood vessel, which becomes a great obstacle to the penetration of a substance from the vascular lumen to the tumor tissue. The drug delivery enhancer of the present invention normalizes abnormal blood vessels and normalizes the vascular permeability in the affected area of a disease involving abnormal blood vessel formation, thereby significantly enhancing the delivery efficiency of a drug used in combination to the affected area.

The substance that activates a lysophospholipid receptor is not limited to a lysophospholipid. A derivative of a lysophospholipid, a precursor of a lysophospholipid, or a derivative thereof may also be suitable as the active ingredient. A lysophospholipid receptor agonist other than these (e.g., a low molecular weight compound, a nucleic acid, a peptide, a protein, an antibody, etc.) may also be suitable as the active ingredient. A known lysophospholipid receptor agonist includes, for example, the LPA4 receptor agonist described in Wong et al. (Assay Drug Dev Technol. 2010 Aug;8(4):459-70. doi:10.1089/adt.2009.0261.). Preferred is a lysophospholipid, a precursor thereof or a derivative thereof.

Examples of the lysophospholipid used in the drug delivery enhancer of the present invention include lysophosphatidic acid (LPA), lysophosphatidylserine (LPS), lysophosphatidylcholine (LPC), lysophosphatidylethanolamine (LPE), lysophosphatidylinositol (LPI), lysophosphatidylglycerol (LPG), sphingosine-1-phosphate (S1P), sphingosylphosphorylcholine (SPC), and platelet activating factor (PAF). The lysophospholipid is not limited to these and other lysophospholipids may also be suitable for the present invention. Preferred are lysophosphatidic acid, lysophosphatidylcholine, and sphingosine-1-phosphate, and more preferred is lysophosphatidic acid. The drug delivery enhancer of the present invention may comprise one or a combination of two or more lysophospholipids. The acyl group of the lysophospholipid used herein is not particularly limited, but is preferably an acyl group of 16 to 22 carbon atoms with a degree of unsaturation of 0 to 6. More preferably, the ratio of the number of carbon atoms to the degree of unsaturation in the acyl group is 16:1, 18:1, 18:2, 18:3, 20:1, 20:2, 20:3, 20:4, 20:5, 22:1, 22:2, 22:3, 22:4, 22:5, or 22:6. The lysophospholipid may be a 1-acyl lysophospholipid or a 2-acyl lysophospholipid. Preferred is a 1-acyl lysophospholipid.

Examples of the precursor of the lysophospholipid include a phosphatidic acid, a phosphatidylserine, a phosphatidylcholine, a phosphatidylethanolamine, a phosphatidylinositol, aphosphatidylglycerol, a sphingomyelin, and a sphingolipid. As is readily understood by a person skilled in the art, these phospholipids are metabolized in a living body to generate lysophospholipids (see, e.g., E. J. Goetzl, S. An, FASEB J. 12, 1589 (1998), and Xie Y, Meier KE. Cell Signal. 2004 Sep;16(9):975-81).

Examples of the derivative of the lysophospholipid include lysophospholipids modified for the purpose of improving the stability in the blood, such as a lysophospholipid modified with a polyethylene glycol (PEG) derivative (a PEGylated lysophospholipid), a lysophospholipid modified with a water-soluble polymer such as a polyglycerol, and a lysophospholipid modified with any given substituent. Examples of the derivative of the lysophospholipid precursor include a lysophospholipid precursor modified with a PEG derivative, a lysophospholipid precursor modified with a water-soluble polymer, and a lysophospholipid precursor modified with any given substituent. The lysophospholipid, the precursor thereof and the derivative thereof may form a salt. The salt is preferably physiologically acceptable. Examples of the physiologically acceptable salt include salts with acids such as hydrochloric acid, sulfuric acid, phosphoric acid, lactic acid, tartaric acid, maleic acid, fumaric acid, oxalic acid, malic acid, citric acid, oleic acid, palmitic acid, nitric acid, phosphoric acid, trifluoroacetic acid, methanesulfonic acid, benzenesulfonic acid, and p-toluenesulfonic acid; salts with hydroxides or carbonates of an alkali metal, such as sodium, potassium and calcium, salts with hydroxides or carbonates of an alkaline earth metal, and salts with aluminum hydroxide or carbonate; and salts with triethylamine, benzylamine, diethanolamine, t-butylamine, dicyclohexylamine, arginine, etc.

The lysophospholipid, the precursor thereof or the derivative thereof used in the present invention can be obtained by known methods, including, for example, (1) chemical synthesis, (2) purification from a biological sample, and (3) enzymatic synthesis. The lysophospholipid, the precursor thereof or the derivative thereof may be a commercially available product. In the case of chemical synthesis, the lysophospholipid, the precursor thereof or the derivative thereof may be produced by combining appropriately modified versions of the methods described in, for example, Comprehensive Organic Transformations: A Guide to Functional Group Preparations, 2nd Edition (Richard C. Larock, John Wiley & Sons Inc, 1999). In the case of purification from a biological sample, the lysophospholipid, the precursor thereof or the derivative thereof may be produced by, for example, obtaining a fraction by gel filtration or other means and purifying the fraction by silica gel chromatography or reversed-phase column chromatography. In the case of enzymatic synthesis, the lysophospholipid, the precursor thereof or the derivative thereof may be produced by using, for example, myeloperoxidase, an oxidase, 12/15-lipoxygenase, or a P450 metabolic enzyme.

The lysophospholipid receptor that is to be regulated by the active ingredient is not particularly limited, and may be a known lysophospholipid receptor or an as yet undiscovered lysophospholipid receptor. Preferred is a lysophospholipid receptor expressed in vascular endothelial cells, and more preferred is a lysophospholipid receptor specifically expressed in vascular endothelial cells. For example, the inventors found that LPAR4 and LPAR6 are specifically expressed in vascular endothelial cells of mouse tumor tissues (see Example 10). The inventors then revealed that LPAR4 is particularly preferred as a target (see Example 11). Thus, the lysophospholipid receptor that is to be regulated by the active ingredient is preferably human LPARs corresponding to mouse LPAR4 and LPAR6.

The diseases involving abnormal blood vessel formation are exemplified by diseases involving the formation of blood vessels with tortuous course and irregular branching in the affected area, diseases involving the formation of blood vessels with irregular luminal structure in the affected area, and diseases involving the formation of blood vessels with increased permeability in the affected area. The diseases involving abnormal blood vessel formation to which an drug is effectively delivered with the aid of the drug delivery enhancer of the present invention are exemplified by, but are not limited to, solid cancers, age-related macular degeneration, rheumatoid arthritis, psoriasis, scleroderma, systemic lupus erythematosus, vasculitis syndrome, vasculo-Behcet's disease, diabetic retinopathy, diabetic nephropathy, arteriosclerosis, chronic arteriosclerosis obliterans, Buerger's disease, pulmonary fibrosis, cerebral infarction, serious infections, and cardiac failure.

Solid cancers have blood vessels with tortuous course and irregular branching, and the blood vessels have irregular luminal structure and excessively increased permeability. Examples of the solid cancers include, but are not limited to, lung cancer, colon cancer, prostate cancer, breast cancer, pancreatic cancer, esophageal cancer, gastric cancer, liver cancer, biliary cancer, spleen cancer, renal cancer, bladder cancer, uterine cancer, ovarian cancer, testis cancer, thyroid cancer, and cerebral tumor. Solid cancers also include a tumor derived from cancerous blood cells. Age-related macular degeneration is a disease that causes the formation of new blood vessels with increased permeability in the choroid. Leakage of blood components from the newly formed blood vessels in the choroid causes retinal edema and subretinal fluid accumulation, leading to visual impairments. Rheumatoid arthritis is a disease that develops inflammation of the synovial membrane in a joint, which induces the formation of new blood vessels with increased permeability. The accumulation of synovial fluid in a joint cavity causes the deformation of the joint and pain. Psoriasis is an inflammatory disease that develops tortuous blood vessels with irregular structure in the stratum corneum of the skin, and is often accompanied by inflammation and hyperkeratosis. Scleroderma is a collagen disease in which inflammation induces the formation of new blood vessels with increased permeability, leading to constant inflammation in the skin. Systemic lupus erythematosus, vasculitis syndrome, and vasculo-Behcet's disease also develop inflammation that induces the formation of new blood vessels with increased permeability. Diabetic retinopathy is a disease in which a constantly high level of blood sugar damages capillary vessels in the retina to make the vessels fragile, and part of the damaged vessels develops swelling leading to abnormal vasculature. When the fragile capillaries are blocked by a clot, the capillaries may form new blood vessels. The newly formed blood vessels are fragile and have increased permeability, and when hemorrhage occurs, it leads to blindness. Diabetic nephropathy is a disease in which a high level of blood sugar damages capillary vessels in the glomerulus, which induces the formation of new blood vessels with increased permeability, resulting in failure of the filtering function of the glomerulus to allow the leakage of serum proteins and other necessary components into urine. Arteriosclerosis is a disease in which the accumulation of cholesterol and other substances in blood vessels forms an atherosclerotic plaque to narrow the vascular lumen, leading to reduction in the blood flow. In the atherosclerotic plaque, many blood vessels with irregular structure are built up, which contribute to the growth of the atherosclerotic plaque. Chronic arteriosclerosis obliterans is a disease in which the large vessels of the extremities are constantly narrowed to cause reduction in the blood flow, and the tissue hypoxia induces the formation of new blood vessels with increased permeability, leading to the development of edema and inflammation. Buerger's disease, also called thromboangiitis obliterans, is a disease with an unknown cause. In Buerger's disease, inflammation of the peripheral arteries induces narrowing of vascular lumens, resulting in a blood flow disorder. Due to intense inflammation of the arteries, endothelial cells in the tunica intima of the damaged arteries form new blood vessels with irregular structure and increased permeability. Pulmonary fibrosis is a disease in which fibrosis occurs in the inflammatory area of the lung due to interstitial pneumonia. In the acute stage, infiltration of inflammatory cells to the stroma of the lung occurs, which induces the build-up of new blood vessels with increased permeability. Cerebral infarction is a disease in which the blockage in the blood vessels in the brain reduces the blood flow and induces the cell death of nerve cells. Cerebral infarction enhances the expression of vascular endothelial growth factor (VEGF) and other factors, which induces the formation of new blood vessels with increased permeability and also increases the permeability of already existing blood vessels, resulting in life-threatening brain edema. Serious infections enhance the expression of inflammatory cytokines. Similarly to the situation in cerebral infarction, these cytokines induce the formation of new blood vessels with increased permeability and also increases the permeability of already existing blood vessels, resulting in life-threatening edema with bleeding from the blood vessels of some organs or of the whole body. Cardiac failure is a disease in which reduction in the systemic blood flow induces tissue hypoxia. Similarly to the situation in cerebral infarction, the tissue hypoxia induces the formation of new blood vessels with increased permeability and also enhances the permeability of already existing blood vessels, resulting in pleural effusion, hepatic congestion, digestive tract edema, or pulmonary congestion.

A therapeutic drug suitable for use in combination with the drug delivery enhancer of the present invention may be a therapeutic drug for any of the diseases described above. Specific examples thereof include anticancer drugs, anti-inflammatory drugs, antipsychotic drugs, sensory system drugs, circulatory system drugs, respiratory drugs, digestive system drugs, endocrinologic and metabolic drugs, renal and urological drugs, vitamin formulations, nutrient formulations, intravenous infusions, electrolyte formulations, hematologic drugs, blood derivatives, immunosuppressive drugs, analgesic drugs, antiallergic drugs, antibiotic drugs, antibacterial drugs, and antiviral drugs. The term "combined use of the drug delivery enhancer and a therapeutic drug for a disease involving abnormal blood vessel formation" herein means that the period of treatment with the drug delivery enhancer of the present invention overlaps with the period of treatment with the therapeutic drug, not requiring simultaneous administration of the two types of drugs.

### Pharmaceutical composition

The present invention provides a pharmaceutical composition for treating a disease involving abnormal blood vessel formation, the composition comprising, as an active ingredient, a substance that activates a lysophospholipid receptor. The drug delivery enhancer of the present invention is preferably provided in the form of a pharmaceutical product. The pharmaceutical composition of the present invention may be used alone or in combination with another drug for treating a disease involving abnormal blood vessel formation. The substance that activates a lysophospholipid receptor is not limited to a lysophospholipid. A derivative of a lysophospholipid, a precursor of a lysophospholipid, or a derivative thereof may also be suitable as the active ingredient. A lysophospholipid receptor agonist other than these (e.g., a low molecular weight compound, a nucleic acid, a peptide, a protein, an antibody, etc.) may also be suitable as the active ingredient. A known lysophospholipid receptor agonist includes, for example, the LPA4 receptor agonist described in Wong et al. (Assay Drug Dev Technol. 2010 Aug;8(4):459-70. doi:10.1089/adt.2009.0261.). Preferred is a lysophospholipid, a precursor thereof or a derivative thereof.

The substance that activates a lysophospholipid receptor, which serves as an active ingredient of the pharmaceutical composition of the present invention, has one or more effects selected from inhibitory effect on tumor growth, inhibitory effect on cancer metastasis, enhancing effect on immunity against a tumor, and inhibitory effect on an increase in vascular permeability. Due to these effects, the pharmaceutical composition of the present invention is suitable to be used alone for the treatment of a disease involving abnormal blood vessel formation. Also due to its effect of normalizing vascular permeability, the pharmaceutical composition of the present invention, when used in combination with another drug for treating a disease involving abnormal blood vessel formation, enhances the delivery of said another drug to the affected area. The disease involving abnormal blood vessel formation as a therapeutic target is as defined above, and non-limiting examples thereof include solid cancers, age-related macular degeneration, articular rheumatism, psoriasis, scleroderma, systemic lupus erythematosus, vasculitis syndrome, vasculo-Behcet's disease, diabetic retinopathy, diabetic nephropathy, arteriosclerosis, chronic arteriosclerosis obliterans, Buerger's disease, pulmonary fibrosis, cerebral infarction, serious infections, and cardiac failure.

The pharmaceutical composition of the present invention can be produced by appropriately mixing the substance that activates a lysophospholipid receptor as an active ingredient with a pharmaceutically acceptable carrier or additive in accordance with a known production method for pharmaceutical preparations (e.g., the methods described in the Japanese pharmacopoeia, etc.). In particular, the pharmaceutical composition may be, for example, an oral preparation or a parenteral preparation, including tablets (including sugar-coated tablets, film-coated tablets, sublingual tablets, orally disintegrating tablets, and buccal tablets), pills, powders, granules, capsules (including soft capsules and microcapsules), troches, syrups, liquids, emulsions, suspensions, controlled-release preparations (e.g., fast-release preparations, sustained release preparations, sustained release microcapsules, etc.), aerosols, films (e.g., orally disintegrating films, oral mucosal adhesive films, etc.), injections (e.g., subcutaneous injections, intravenous injections, intramuscular injections, intraperitoneal injections, etc.), intravenous infusions, transdermal preparations, ointments, lotions, patches, suppositories (e.g., rectal suppositories, vaginal suppositories, etc.), pellets, transnasal preparations, transpulmonary preparations (inhalants), and eye drops. The amount of the carrier or additive to be added can be determined as appropriate based on the range typically used in the pharmaceutical field. The carrier or additive that may be added is not particularly limited and examples thereof include various types of carriers such as water, physiological saline, other aqueous solvents, and aqueous or oily vehicles; and various types of additives such as excipients, binders, pH adjusters, disintegrants, absorption promoters, lubricants, colorants, flavors and fragrances.

Examples of the additives that may be added to tablets, capsules, etc. include binders such as gelatin, corn starch, tragacanth, and gum arabic; excipients such as crystalline cellulose; swelling agents such as corn starch, gelatin, and alginic acid; lubricants such as magnesium stearate; sweeteners such as sucrose, lactose, and saccharin; and flavors such as peppermint flavor, wintergreen oil, and cherry flavor. When the unit dosage form is a capsule, a liquid carrier such as oils and fats can be further added in addition to the above types of materials. A sterile composition for injection can be prepared in accordance with a usual formulation procedure (for example, by dissolving or suspending the active ingredient in a solvent such as water for injection or a natural vegetable oil). Aqueous liquids for injection that may be used are, for example, physiological saline and an isotonic solution containing glucose and/or other auxiliary substances (for example, D-sorbitol, D-mannitol, sodium chloride, etc.). The aqueous liquids for injection may be used in combination with an appropriate solubilizer, such as alcohols (ethanol etc.), polyalcohols (propylene glycol, polyethylene glycol, etc.), and nonionic surfactants (polysorbate 80™, HCO-50, etc.). Oily liquids that may be used are, for example, sesame oil and soybean oil. The oily liquids may be used in combination with a solubilizer such as benzyl benzoate and benzyl alcohol. Other additives that may be added are, for example, buffering agents (e.g., a phosphate buffer, a sodium acetate buffer, etc.), soothing agents (e.g., benzalkonium chloride, procaine hydrochloride, etc.), stabilizers (e.g., human serum albumin, polyethylene glycol, etc.), preservatives (e.g., benzyl alcohol, phenol, etc.) and antioxidants.

The lysophospholipid or a derivative thereof, which serves an active ingredient of the pharmaceutical composition of the present invention, is a substance found in a living body. Hence, the pharmaceutical composition of the present invention has low toxicity to humans and other mammals (e.g., rats, mice, rabbits, sheep, pig, cattle, cats, dogs, monkeys, etc.), and can be administered safely.

In cases where the lysophospholipid or a derivative thereof is used as an active ingredient of the pharmaceutical composition of the present invention, the amount of the active ingredient contained in the pharmaceutical composition may vary with the dosage form, the administration method, the carrier to be used, etc., but is usually 0.01 to 100% (w/w), preferably 0.1 to 95% (w/w), relative to the total amount of the pharmaceutical composition. The pharmaceutical composition of the present invention containing the lysophospholipid or a derivative thereof in such an amount can be produced in accordance with a conventional method.

The dosage varies with the subject, the symptom, the route of administration, etc., but in general, the dosage for oral administration to a human with a body weight of about 60 kg is about 0.01 to 1000 mg per day, preferably about 0.1 to 100 mg per day, and more preferably about 0.5 to 500 mg per day.

The dose for single parenteral administration varies with the conditions of the patient, the symptom, the administration method, etc. In the case of, for example, an intravenous injection, the dose is, for example, usually about 0.01 to 100 mg per kg of body weight, preferably about 0.01 to 50 mg per kg of body weight, and more preferably about 0.01 to 20 mg per kg of body weight. The total daily dosage may be administered in a single dose or in divided doses.

Preferably, the pharmaceutical composition of the present invention is used in combination with another drug for treating a disease involving abnormal blood vessel formation. Said another drug for treating a disease involving abnormal blood vessel formation is not particularly limited, and such a drug suitable for use in combination may be, for example, a known drug that is usually used for the treatment of any of the above-exemplified diseases involving abnormal blood vessel formation. Specific examples thereof include anticancer drugs, anti-inflammatory drugs, antipsychotic drugs, sensory system drugs, circulatory system drugs, respiratory drugs, digestive system drugs, endocrinologic and metabolic drugs, renal and urological drugs, vitamin formulations, nutrient formulations, intravenous infusions, electrolyte formulations, hematologic drugs, blood derivatives, immunosuppressive drugs, analgesic drugs, antiallergic drugs, antibiotic drugs, antibacterial drugs, and antiviral drugs. More preferably, the pharmaceutical composition is used in combination with an anticancer drug for the treatment of a solid cancer and the inhibition of metastasis. Combined administration of the pharmaceutical composition of the present invention with said another drug enhances the delivery of the drug to the affected area of a disease involving abnormal blood vessel formation. Thus, the dosage of the drug can be reduced, which may lead to reduction in adverse effects caused by the drug. The reduction in the dosage of the drug also may satisfy social demands such as the reduction of the medical costs.

The anticancer drug to be used in combination with the pharmaceutical composition of the present invention is not particularly limited, but preferred are a chemotherapeutic drug, an immunotherapeutic drug, or a hormone therapy drug. These anticancer drugs may be in the form of liposomal formulations. These cancer therapeutic drugs may be in the form of nucleic acid formulations or antibody formulations.

The chemotherapeutic drug is not particularly limited and examples thereof include alkylating agents such as nitrogen mustard, nitrogen mustard N-oxide hydrochloride, chlorambucil, cyclophosphamide, ifosfamide, thiotepa, carboquone, improsulfan tosilate, busulfan, nimustine hydrochloride, mitobronitol, melphalan, dacarbazine, ranimustine, estramustine phosphate sodium, triethylenemelamine, carmustine, lomustine, streptozocin, pipobroman, ethoglucid, carboplatin, cisplatin, miboplatin, nedaplatin, oxaliplatin, altretamine, ambamustine, dibrospidium chloride, fotemustine, prednimustine, pumitepa, Ribomustin, temozolomide, treosulfan, trofosfamide, zinostatin stimalamer, adozelesin, cystemustine and bizelesin; antimetabolites such as mercaptopurine, 6-mercaptopurine riboside, thioinosine, methotrexate, pemetrexed, enocitabine, cytarabine, cytarabine ocfosfate, ancitabine hydrochloride, 5-FU and its derivatives (e.g., fluorouracil, tegafur, UFT, doxifluridine, carmofur, galocitabine, emitefur, capecitabine, etc.), aminopterin, nelzarabine, leucovorin calcium, Tabloid, butocin, calcium folinate, calcium levofolinate, cladribine, emitefur, fludarabine, gemcitabine, hydroxycarbamide, pentostatin, piritrexim, idoxuridine, mitoguazone, tiazofurin, ambamustine and bendamustine; anticancer antibiotics such as actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarkomycin, carzinophilin, mitotane, zorubicin hydrochloride, mitoxantrone hydrochloride and idarubicin hydrochloride; and plant-derived anticancer drugs such as etoposide, etoposide phosphate, vinblastine sulfate, vincristine sulfate, vindesine sulfate, teniposide, paclitaxel, docetaxel, vinorelbine, irinotecan, and irinotecan hydrochloride.

The immunotherapeutic drug is not particularly limited and examples thereof include Picibanil, Krestin, sizofiran, lentinan, ubenimex, interferons, interleukins, macrophage colony-stimulating factor, granulocyte colony-stimulating factor, erythropoietin, lymphotoxins, BCG vaccine, Corynebacterium parvum, levamisole, polysaccharide K, procodazole, ipilimumab, nivolumab, ramucirumab, ofatumumab, panitumumab, pembrolizumab, obinutuzumab, trastuzumab emtansine, tocilizumab, bevacizumab, trastuzumab, siltuximab, cetuximab, infliximab, and rituximab.

The hormone therapy drug is not particularly limited and examples thereof include fosfestrol, diethylstilbestrol, chlorotrianisene, medroxyprogesterone acetate, megestrol acetate, chlormadinone acetate, cyproterone acetate, danazol, allylestrenol, gestrinone, mepartricin, raloxifene, ormeloxifene, levormeloxifene, antiestrogens (e.g., tamoxifen citrate, toremifene citrate, etc.), birth-control pills, mepitiostane, testololactone, aminoglutethimide, LH-RH agonists (e.g., goserelin acetate, buserelin, leuprorelin, etc.), droloxifene, epitiostanol, ethinylestradiol sulfonate, aromatase inhibitors (e.g., fadrozole hydrochloride, anastrozole, letrozole, exemestane, vorozole, formestane, etc.), antiandrogens (e.g., flutamide, bicalutamide, nilutamide, etc.), 5α-reductase inhibitors (e.g., finasteride, epristeride, etc.), corticosteroids (e.g., dexamethasone, prednisolone, betamethasone, triamcinolone, etc.) and androgen synthesis inhibitors (e.g., abiraterone, etc.).

When the pharmaceutical composition of the present invention is used in combination with another drug for treating a disease involving abnormal blood vessel formation, the composition and the drug may be simultaneously administered to the subject or separately administered to the subject with a certain time interval between each administration. The term "use in combination" herein means that the period of treatment with the drug delivery enhancer of the present invention overlaps with the period of treatment with the therapeutic drug, not requiring simultaneous administration of the two types of drugs. The dosage of said another drug for treating a disease involving abnormal blood vessel formation may be determined in accordance with its clinically approved dosage, and is appropriately selected depending on the subject, the age and body weight of the subject, the symptom, the duration of the administration, the dosage form, the administration method, the combination of the drugs, etc.

### Screening method

The present invention also provides a method for screening for a therapeutic drug for a disease involving abnormal blood vessel formation. The screening method of the present invention comprises selecting a substance that activates a lysophospholipid receptor specifically expressed in vascular endothelial cells of the affected area (a lysophospholipid receptor agonist).

Examples of the lysophospholipid receptor include, but are not limited to, lysophosphatidic acid receptors (LPARs), lysophosphatidylserine receptors (LPSRs), lysophosphatidylcholine receptors (LPCRs), lysophosphatidylethanolamine receptors (LPERs), lysophosphatidylinositol receptors (LPIRs), lysophosphatidylglycerol receptors (LPGRs), sphingosine-1-phosphate receptors (S1PRs), sphingosylphosphorylcholine receptors (SPCRs), and platelet activating factor receptors (PAFRs). Preferred are lysophosphatidic acid receptors (LPARs).

The lysophospholipid receptor that is specifically expressed in vascular endothelial cells of the affected area is not particularly limited. Any lysophospholipid receptor that is specifically expressed in vascular endothelial cells of the affected area is suitable as a target for the screening method, including as yet undiscovered lysophospholipid receptors. For example, the inventors found that LPAR4 and LPAR6 are specifically expressed in vascular endothelial cells of mouse tumor tissues (see Example 10). The inventors then revealed that LPAR4 is particularly preferred as a target (see Example 11). Thus, the receptor as a target of the screening method of the present invention is preferably human LPARs corresponding to mouse LPAR4 and LPAR6.

The screening method of the present invention may comprise, for example, the steps of contacting test substances with a lysophospholipid receptor specifically expressed in vascular endothelial cells of the affected area of a disease involving abnormal blood vessel formation, measuring the levels of the activation of the lysophospholipid receptor, and selecting the test substance that activates the lysophospholipid receptor. The substance selected by the screening method of the present invention is useful as an active ingredient of the drug delivery enhancer of the present invention. The substance selected by the screening method of the present invention is also useful as an active ingredient of the pharmaceutical composition of the present invention for treating a disease involving abnormal blood vessel formation.

The present invention also includes the following.

A method for enhancing the delivery of a therapeutic drug for a disease involving abnormal blood vessel formation to an affected area, the method comprising administering an effective amount of a lysophospholipid, a precursor thereof or a derivative thereof to a mammal, and administering an effective amount of a therapeutic drug for a disease involving abnormal blood vessel formation.

A method for treating a disease involving abnormal blood vessel formation, the method comprising administering an effective amount of a lysophospholipid, a precursor thereof or a derivative thereof to a mammal, and administering an effective amount of a therapeutic drug for a disease involving abnormal blood vessel formation.

A method for treating a solid cancer, the method comprising administering an effective amount of a lysophospholipid, a precursor thereof or a derivative thereof to a mammal.

A method for inhibiting cancer metastasis, the method comprising administering an effective amount of a lysophospholipid, a precursor thereof or a derivative thereof to a mammal.

A lysophospholipid, a precursor thereof or a derivative thereof for use in the enhancement of the delivery of a therapeutic drug for a disease involving abnormal blood vessel formation to an affected area.

A lysophospholipid, a precursor thereof or a derivative thereof for use in the treatment of a disease involving abnormal blood vessel formation.

A lysophospholipid, a precursor thereof or a derivative thereof for use in the treatment of a solid cancer.

A lysophospholipid, a precursor thereof or a derivative thereof for use in the inhibition of cancer metastasis.

Use of a lysophospholipid, a precursor thereof or a derivative thereof for the production of a drug delivery enhancer for enhancing the delivery of a therapeutic drug for a disease involving abnormal blood vessel formation to an affected area.

Use of a lysophospholipid, a precursor thereof or a derivative thereof for the production of a therapeutic drug for a disease involving abnormal blood vessel formation.

Use of a lysophospholipid, a precursor thereof or a derivative thereof for the production of a therapeutic drug for a solid cancer.

Use of a lysophospholipid, a precursor thereof or a derivative thereof for the production of an inhibitor of cancer metastasis.

### EXAMPLES

The present invention will be described in more detail below in reference to Examples, but is not limited to these Examples. The term "%" herein means % by mass unless otherwise specified.

### Example 1: Structural changes in tumor blood vessels after administration of lysophosphatidic acid (LPA)

To examine LPA-induced structural changes in blood vessels, a mouse cancer cell line was subcutaneously inoculated into mice to establish a tumor, followed by administration of LPA.

### (1) Experimental method

Lewis lung cancer cell line (hereinafter called LLC cells) was used as the mouse cancer cell line. LLC cells (1 × 10⁶ cells in 100 µL PBS per animal) were subcutaneously injected into C57BL/6 NCrSlc mice aged 8 weeks (females, SLC, Inc.).

The LPA used was 18:1 LPA (Avanti Polar Lipids, Inc.). A 10 mM LPA stock solution was prepared using 50% ethanol and the solution was stored at -30°C. Before use, the LPA stock solution was thawed and homogenized with an ultrasonic cleaner (SND Co., Ltd.) for 1 minute. The solution was diluted in PBS before administration so that the concentration of LPA was 3 mg/kg in 100 µL PBS. The prepared solution was used for LPA administration.

Day 9 post-inoculation of LLC cells, mice that developed a tumor with a volume of 60 to 80 mm³ (volume = length × width × height × 0.5) were selected and used for the test. Four groups each of three mice were provided: control group, LPA/6 hr group, LPA/12 hr group and LPA/24 hr group. LPA was intraperitoneally administered to the mice of the LPA groups at a dose of 3 mg/kg in 100 µL PBS. The tumors were harvested before LPA administration (control group), and at 6, 12 and 24 hours after LPA administration. The tumors were immersed in 4% paraformaldehyde (PFA)/PBS and shaken at 4°C overnight for fixation. The tumors were washed with cold PBS (4°C). Washing was continued for 6 hours, and PBS was renewed every 30 minutes. The tumors were immersed in 15% sucrose/PBS and shaken at 4°C for 3 hours. The tumors were then immersed in 30% sucrose/PBS and shaken at 4°C for 3 hours. The tumors were embedded in O.C.T. compound (Tissue-Tek) and frozen at -80°C for 3 days or longer.

The tumors embedded in O.C.T. compound were sectioned at 40 µm with a cryostat (Leica). The sections were placed on glass slides, and air-dried for 2 hours with a drier. The sections were encircled with a liquid blocker. The glass slides were placed in a staining jar, and washed with PBS at room temperature for 10 minutes to wash off O.C.T. compound. The sections were post-fixed in 4% PFA/PBS at room temperature for 10 minutes, and washed with PBS at room temperature for 10 minutes. A few drops of blocking solution (5% normal goat serum, 1% BSA and 2% skim milk in PBS) were applied to the sections, and the sections were blocked at room temperature for 20 minutes. Anti-mouse CD31, Purified Hamster Anti-PECAM-1 (MAB1398Z, Millipore) antibody as a primary antibody was diluted to 200-fold in blocking solution and a few drops of the antibody were applied to the sections. The sections were incubated at 4°C overnight. The sections were washed five times with PBS containing Tween 20 (PBST) each for 10 minutes and further with PBS for 10 minutes. Alexa Fluor 488 Goat Anti-Hamster IgG (Jackson ImmunoResearch Laboratories) as a secondary antibody was diluted to 400-fold in blocking solution and a few drops of the antibody were applied to the sections. The sections were incubated under light-shielding for 2 hours. The sections were washed five times with PBST each for 10 minutes. Several drops of Vectashield (Vector Laboratories Inc.) were applied to the sections and the sections were covered with glass coverslips. The sections were observed and photographed under a confocal laser microscope (Leica). The vessel length was measured in the photographs using the vascular structure analysis software AngioTool.

### (2) Results

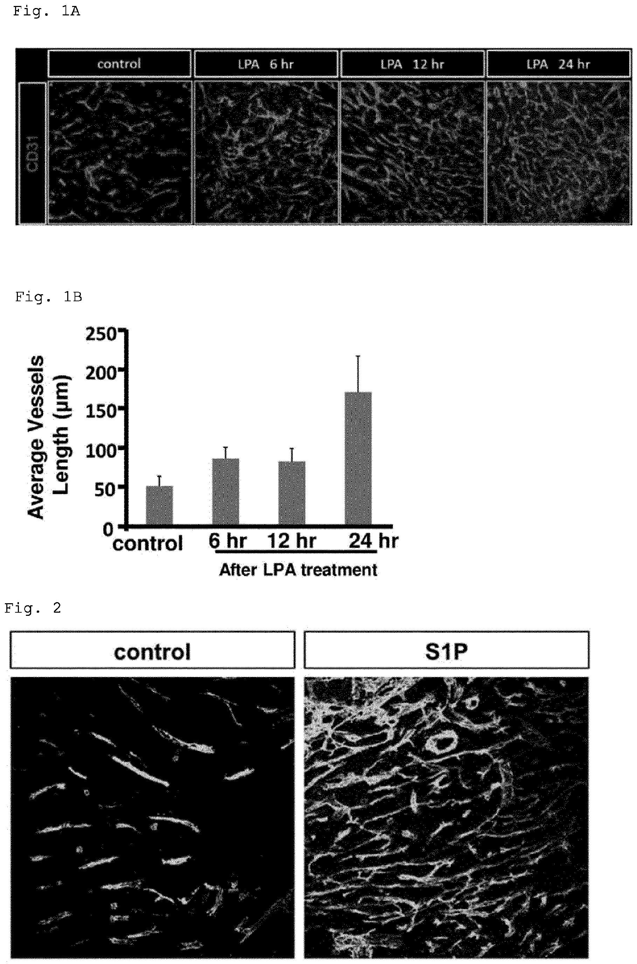

The results are shown in Figs. 1A and 1B. Fig. 1A shows the photographs of the specimens observed under a confocal laser microscope. Vascular endothelial cells were stained with fluorescent green, and are shown with white color in the attached figures. Fig. 1B is a chart showing the average length of blood vessels in each group quantified with the vascular structure analysis software AngioTool. As shown in Fig. 1A, the tumor blood vessels before LPA administration (control) were discontinuous and had a sparse network. At 6 hours after administration, the connection of the blood vessels was observed. At 12 and 24 hours after administration, the formation of a fine vascular network similar to that in normal tissues was observed. Fig. 1B numerically shows that the length of blood vessels was increased by LPA administration. These results indicated that the formation of a network of tumor blood vessels was induced in a short period of time after LPA administration. Hence, the formation of a network of tumor blood vessels by LPA may not be attributed to the proliferation of vascular endothelial cells, but are attributed more to the spreading, adhesion and lumen formation of vascular endothelial cells. Although the results are not shown here, similar results were obtained in the experiments using cancer cell lines other than LLC cells, such as colon 26 colon cancer cells or B16 melanoma cells.

### Example 2: Structural changes in tumor blood vessels after administration of sphingosine-1-phosphate (S1P)

Using another lysophospholipid S1P, an investigation was performed to examine whether the formation of a network of tumor blood vessels is induced by S1P in the same manner as in induction by LPA.

### (1) Experimental method

LLC cells were subcutaneously inoculated into C57BL/6 NCrSlc mice aged 8 weeks (females, SLC, Inc.) in the same manner as in Example 1. S1P (Avanti Polar Lipids, Inc.) was dissolved in PBS at 10 mM and the solution was stored at -30°C as a stock solution. Before use, the stock solution was thawed and homogenized with an ultrasonic cleaner (SND Co., Ltd.) for 1 minute. The solution was diluted in PBS before administration so that the concentration of S1P was 0.3 mg/kg in 100 µL PBS. The prepared solution was used for S1P administration.

Mice on day 9 post-inoculation of LLC cells (individuals with a tumor volume of 60 to 80 mm³) were subjected to the experiment. The mice were divided into two groups: control group and S1P group (n = 3). From the day of grouping, S1P was administered to the tail vein of the S1P group mice at a dose of 0.3 mg/kg in 100 µL PBS once daily for consecutive three days. To the control group mice, PBS (100 µL) was administered instead of S1P. At 24 hours after the final administration, the tumors were harvested from the mice, and the specimens of tumor blood vessels were prepared in the same manner as in Example 1. The specimens were observed and photographed under a confocal laser microscope (Leica).

### (2) Results

The results are shown in Fig. 2. The same as in the case of the administration of LPA, the formation of a network of tumor blood vessels was induced by the administration of S1P. The results revealed that the normalization of tumor blood vessels by the induction of a network formation is achieved not only by LPA but also by other lysophospholipids.

### Example 3: Structural changes in lumen of tumor blood vessels after LPA administration

### (1) Experimental method

LLC cells were subcutaneously inoculated into C57BL/6 NCrSlc mice aged 8 weeks (females, SLC, Inc.) in the same manner as in Example 1. An LPA administration solution was prepared in the same manner as in Example 1. Mice on day 9 post-inoculation of LLC cells (individuals with a tumor volume of 60 to 80 mm³) were subjected to the experiment. The mice were divided into two groups: control group and LPA group (n = 3). LPA (3 mg/kg in 10 µL PBS) or PBS (100 µL) was administered intraperitoneally. At 24 hours after LPA or PBS administration, mice were perfusion fixed under anesthesia with pentobarbital (Kyoritsu Seiyaku Corporation). The fixative used was 0.1 M phosphate buffer (pH 7.4) containing 2% formaldehyde and 2.5% glutaraldehyde. Tumors were then harvested and immersed in the same fixative as that used for perfusion and shaken at 4°C overnight. The specimens were further immersed in 0.1 M phosphate buffer (pH 7.4) containing 1% osmium tetroxide and 0.5% potassium ferrocyanide. The specimens were dehydrated in an ascending series of ethanol, then the alcohol was replaced with t-butyl alcohol and the specimens were freeze-dried. Osmium tetroxide was deposited on the specimens, and the intraluminal surface of the blood vessels was observed in an S-4800 scanning electron microscope (Hitachi High-Technologies Corporation).

### (2) Results

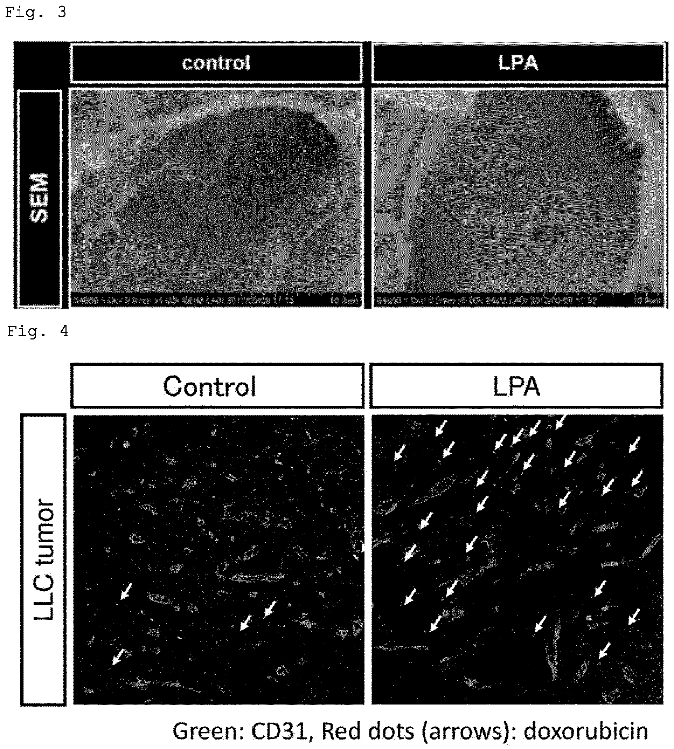

The results are shown in Fig. 3. As is apparent from Fig. 3, filopodia protruding from the intraluminal surface of the blood vessels were observed and the surface of the lumen was very rough in the control group. However, after LPA administration, the intraluminal surface of the blood vessels was very smooth. The results indicated that LPA administration improves the blood circulation in tumors.

### Example 4: Improvement of drug delivery via tumor blood vessels to tumor tissues after LPA administration

As is commonly known, the blood flow in tumors is poor and the vascular permeability is excessively increased. As a result, the fluid pressure in the tumor stroma is increased, leading to no difference in osmotic pressure between the tumor stroma and the blood vessels, which becomes a great obstacle to the penetration of a substance from the vascular lumen to the tumor tissues. Based on the above experimental results showing that LPA administration induces the formation of a dense vascular network in tumors and the formation of a smooth intraluminal surface, it was expected that LPA administration improves drug penetration through tumor blood vessels. To examine drug penetration in tumors after LPA administration, the following experiments were conducted.

### (1) Experimental method

LLC cells were subcutaneously inoculated into C57BL/6 NCrSlc mice aged 8 weeks (females, SLC, Inc.) in the same manner as in Example 1. An LPA administration solution was prepared in the same manner as in Example 1. On day 11 post-inoculation of LLC cells, mice with a tumor volume of 100 to 120 mm³ were selected. The mice were divided into two groups: control group and LPA group (n = 3). LPA (3 mg/kg in 100 µL PBS) or PBS (100 µL) was administered intraperitoneally. At 24 hours after LPA or PBS administration, doxorubicin (doxorubicin hydrochloride, Nippon Kayaku Co., Ltd.) was administered to the tail vein of the mice at a dose of 1.5 mg/kg under anesthesia with pentobarbital. The doxorubicin was prepared as a solution by dissolving and diluting the doxorubicin hydrochloride in physiological saline (Otsuka Pharmaceutical Co., Ltd.) to a concentration of 1.5 mg/kg and homogenizing the solution with an ultrasonic cleaner for 1 minute before administration. Doxorubicin is an autofluorescent anticancer drug that can be detected at an excitation wavelength of 480 nm and a measurement wavelength of 575 nm. At 20 minutes after administration of doxorubicin, the tumors were harvested from the mice, and the tumor specimens were prepared in the same manner as in Example 1 except that the thickness of the sections was 20 µm. The specimens were observed and photographed under a confocal laser microscope (Leica).

### (2) Results

The results are shown in Fig. 4. In Fig. 4, the arrows indicate the red fluorescent signals of doxorubicin. Vascular endothelial cells emit green fluorescence due to anti-CD31 antibody. As is apparent from Fig. 4, delivery of doxorubicin from the blood vessels to tumors in the deep lobe was observed in the tumors of the LPA administration group, but almost no penetration of doxorubicin into tumors was observed in the control group.

### Example 5: Effects of normalization of tumor blood vessels by LPA on delivery of drugs with different molecular weights

The above experiment confirmed that the anticancer drug delivery is improved as a result of the normalization of tumor blood vessels by administration of the lysophospholipid. To examine whether this improvement is also effective to the delivery of low and high molecular weight substances, delivery of 70 kDa and 2000 kDa dextrans was investigated.

### (1) Experimental method

LLC cells were subcutaneously inoculated into C57BL/6 NCrSlc mice aged 8 weeks (females, SLC, Inc.) in the same manner as in Example 1. An LPA administration solution was prepared in the same manner as in Example 1. On day 11 post-inoculation of LLC cells, mice with a tumor volume of 100 to 120 mm³ were selected. The mice were divided into four groups: control and LPA groups with administration of 70 kDa dextran, and control and LPA groups with administration of 2000 kDa dextran (n = 3) . LPA (3 mg/kg in 100 µL PBS) or PBS (100 µL) was administered intraperitoneally. At 24 hours after LPA or PBS administration, each type of dextran was administered to the tail vein of the mice under anesthesia with pentobarbital. The dextrans used (70 kDa and 2000 kDa) were FITC-labeled dextrans (Sigma). Each type of dextran was prepared as a solution by dissolving and diluting the FITC-labeled dextran in PBS to a concentration of 5 mg/mL, and 100 µL of the solution was used for the administration. At 30 minutes after administration of each type of dextran, the tumors were harvested from the mice, and the tumor specimens were prepared in the same manner as in Example 4. The specimens were observed and photographed under a confocal laser microscope (Leica).

### (2) Results

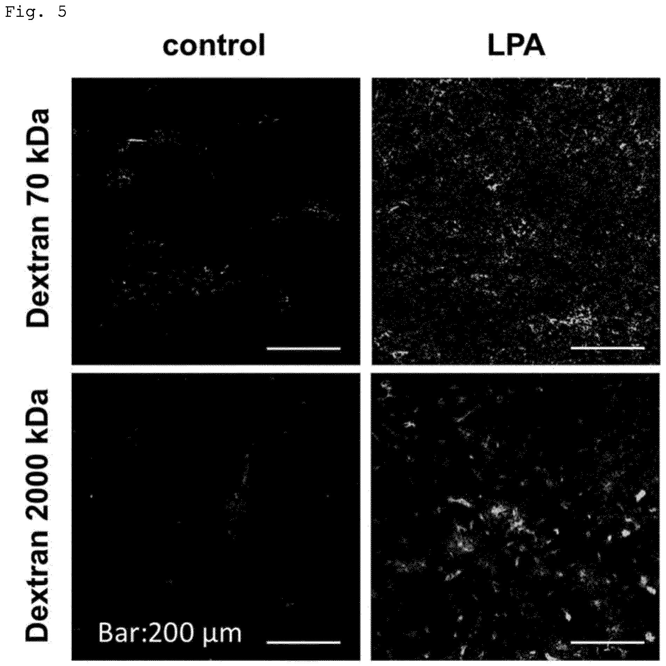

The results are shown in Fig. 5. The delivery of 70 kDa and 2000 kDa dextrans to tumors in the deep lobe was observed in the LPA administration groups, but almost no penetration of the dextrans into tumors was observed in the control groups. The results indicated that the normalization of the vascular permeability of tumor blood vessels by LPA administration effectively improves the delivery of low molecular weight compounds as well as high molecular weight compounds (e.g., nucleic acids, antibodies, etc.) to tumors in the deep lobe.

### Example 6: Study of combination cancer therapy with LPA and anticancer drug

Based on the above results indicating that LPA administration effectively improves the delivery of an anticancer drug to tumors in the deep lobe, a combination cancer therapy of LPA and an anticancer drug was tested.

### 6-1 Effects of combined administration of LPA and 5-FU on LLC cells

### (1) Experimental method

LLC cells were subcutaneously inoculated into C57BL/6 NCrSlc mice aged 8 weeks in the same manner as in Example 1. An LPA administration solution was prepared in the same manner as in Example 1. The anticancer drug used was 5-FU (Kyowa Hakko Kirin Co., Ltd.). The 5-FU was prepared as a solution in physiological saline (Otsuka Pharmaceutical Co., Ltd.). On day 7 post-inoculation of LLC cells, mice with a tumor volume of 30 to 50 mm³ were selected and subjected to the test. The mice were divided into four groups: control group, 5-FU group, LPA group and 5-FU/LPA group (n = 3). LPA (3 mg/kg in 100 µL PBS) or 5-FU (100 mg/kg in 100 µL PBS) or PBS (100 µL) was administered intraperitoneally. To 5-FU/LPA group, both LPA and 5-FU were administered intraperitoneally. LPA and PBS were administered once daily for consecutive seven days. 5-FU was administered twice in total, once a week for two weeks (on days 7 and 14 post-inoculation). The tumor size was measured over time after start of administration. The tumor volume was calculated by the formula: volume = length × width × height × 0.5. Mice at 2 weeks after start of administration were photographed with a digital camera under anesthesia with pentobarbital. The tumors were harvested and photographed with a digital camera.

### (2) Results

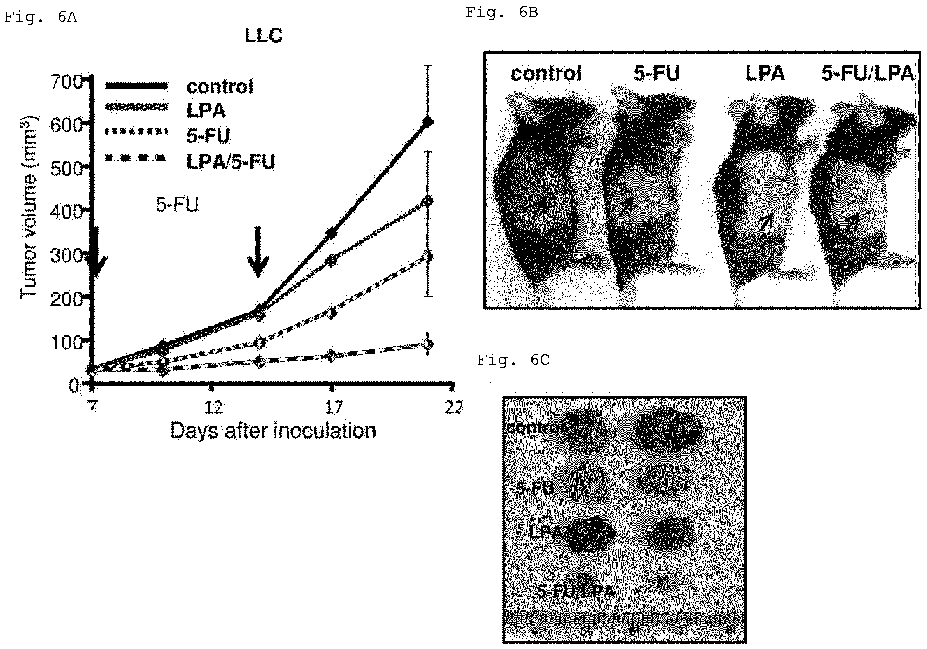

The results are shown in Figs. 6A to 6C. Fig. 6A shows the changes in tumor volume with time in each group. Fig. 6B is a photograph of the appearance of a representative mouse from each group. Fig. 6C is a photograph of the tumors harvested from a representative mouse from each group. As is apparent from Figs. 6A to 6C, combined administration of 5-FU and LPA more markedly inhibited the tumor growth than administration of 5-FU alone. The administration of LPA alone, the tumors appeared reddish, indicating an increase in the blood flow. The administration of LPA alone had some inhibitory effects on tumor growth as compared with the control.

### 6-2 Effects of combined administration of LPA and 5-FU on melanoma cells

### (1) Experimental method

Mouse melanoma B16-BL6 cells were used. B16-BL6 cells (1 × 10⁶ cells in 100 µL PBS per animal) were subcutaneously injected into C57BL/6 NCrSlc mice aged 8 weeks. On day 7 post-inoculation of LLC cells, mice with a tumor volume of 30 to 50 mm³ were selected and subjected to the test. The experiment was performed in the same manner as in 6-1.

### (2) Experimental results

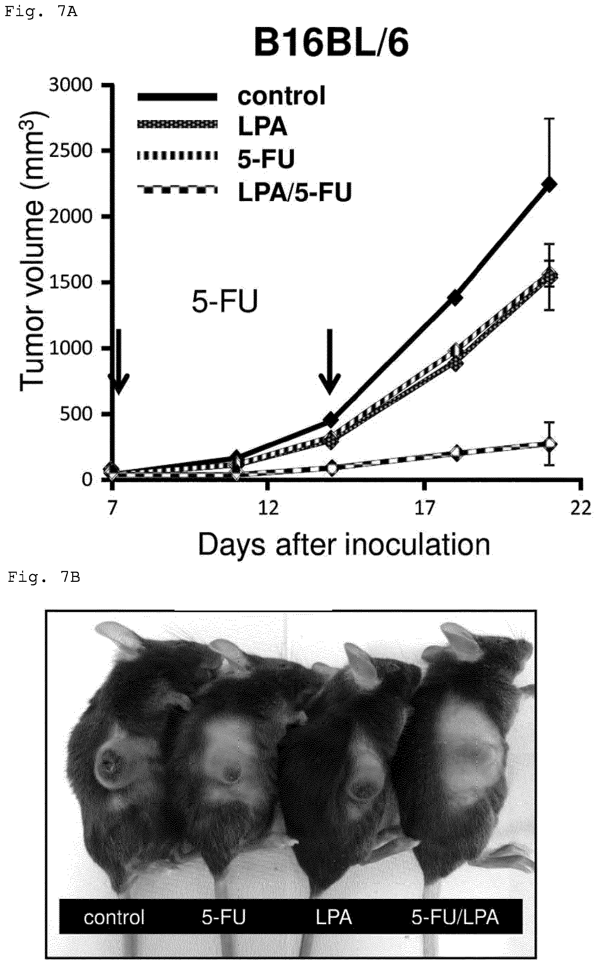

The results are shown in Figs. 7A and 7B. Fig. 7A shows the changes in tumor volume with time in each group. Fig. 7B is a photograph of the appearance of a representative mouse from each group. As is apparent from Figs. 7A and 7B, combined administration of 5-FU and LPA more markedly inhibited tumor growth than administration of 5-FU alone. The administration of LPA alone had some inhibitory effects on tumor growth as compared with the control.

### 6-3 Effects of combined administration of LPA and 5-FU on colon cancer cells

### (1) Experimental method

Mouse colon cancer colon 26 cells were used. Colon 26 cells (1 × 10⁶ cells in 100 µL PBS per animal) were subcutaneously injected into BALB/c mice aged 8 weeks (females, SLC, Inc.). On day 7 post-inoculation of LLC cells, mice with a tumor volume of 30 to 50 mm³ were selected and subjected to the test. The experiment was performed in the same manner as in 6-1.

### (2) Experimental results

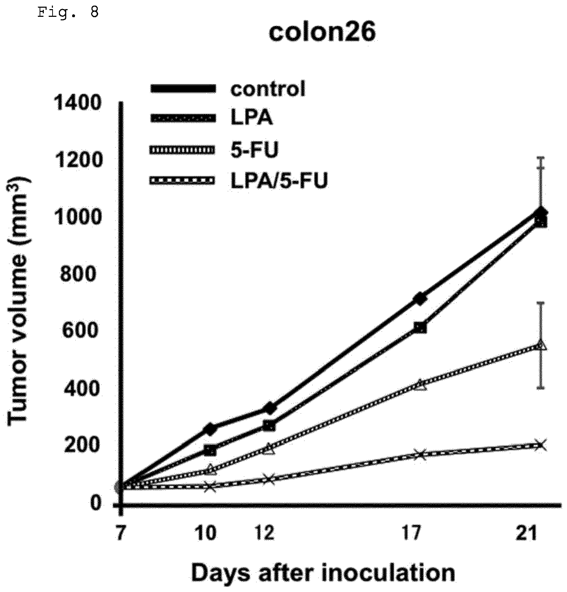

Fig. 8 shows the changes in tumor volume with time in each group. As is apparent from Fig. 8, combined administration of 5-FU and LPA more markedly inhibited tumor growth than administration of 5-FU alone. The administration of LPA alone had some inhibitory effects on tumor growth as compared with the control, up to day 17 post-inoculation.

### 6-4 Effects of combined administration of LPA and oxaliplatin on colon cancer cells

### (1) Experimental method

The experiment was performed in the same manner as in 6-3 except that oxaliplatin was used instead of 5-FU. The oxaliplatin (COSMO BIO Co., Ltd.) was prepared as a solution in physiological saline (Otsuka Pharmaceutical Co., Ltd.) and was intraperitoneally administered at a dose of 1.5 mg/kg in 100 µL PBS.

### (2) Results

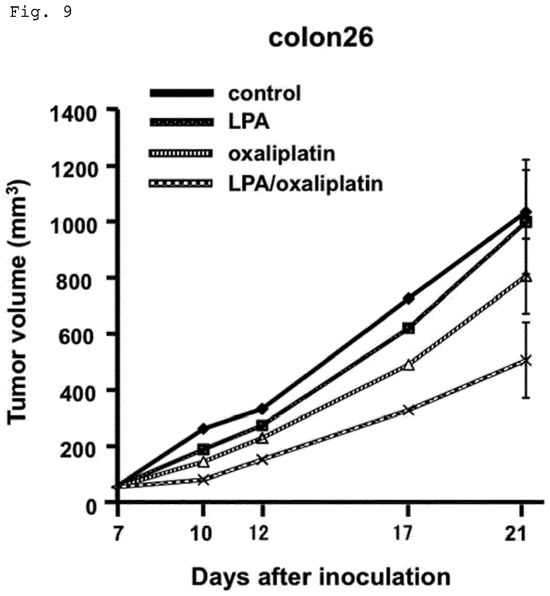

Fig. 9 shows the changes in tumor volume with time in each group. As is apparent from Fig. 9, combined administration of oxaliplatin and LPA more markedly inhibited tumor growth than administration of oxaliplatin alone. The administration of LPA alone had some inhibitory effects on tumor growth as compared with the control, up to day 17 post-inoculation. The results indicated that the synergistic effect by the combined administration of LPA and an anticancer drug is not limited to the case where the anticancer drug is 5-FU.

The overall results suggested that the effect of LPA on tumor blood vessels is effective for cancer tissues derived from any type of cancer cells.

Administration of LPA alone inhibited the growth of all the cell-derived tumors. This may have been because the LPA-induced improvement of the vascular permeability of tumor blood vessels allowed cancer-attacking immune cells to infiltrate the tumor tissues, and in turn the immune cells, including natural killer cells and CD8-positive T cells, attacked cancer cells and induced cell death. The improvement of the vascular permeability may have also induced a predominant increase of M1 macrophages, which have anti-tumor activity. The experimental results suggested that the LPA-induced improvement of the blood flow in tumor blood vessels enhances immunity against a tumor.

### Example 7: Induction of cell-cell adhesion of vascular endothelial cells by LPA administration

In Example 3, the tumor blood vessels of the LPA administration group had a smooth intraluminal surface with no gap. This suggested that the vascular endothelial cell-cell adhesion was tightened in the LPA administration group. Based on this conception, LPA was administered and analysis was performed on the expression level of VE-cadherin in tumor blood vessels. VE-cadherin is a molecule that induces cell-cell adhesion of vascular endothelial cells.

### (1) Experimental method

LLC cells were subcutaneously inoculated into C57BL/6 NCrSlc mice aged 8 weeks (females, SLC, Inc.) in the same manner as in Example 1. An LPA administration solution was prepared in the same manner as in Example 1. Mice on day 9 post-inoculation of LLC cells (individuals with a tumor volume of 60 to 80 mm³) were subjected to the experiment. The mice were divided into two groups: control group and LPA group (n = 3). LPA was intraperitoneally administered to the mice of the LPA group at a dose of 3 mg/kg in 100 µL PBS. The tumors of the control group were harvested just after grouping, and the tumors of the LPA group were harvested at 24 hours after LPA administration. The tumor tissue specimens were immunostained in the same manner as in Example 1 except that the primary antibody and the secondary antibody were changed. The primary antibody was Purified Goat Anti-mouse VE-cadherin (BD pharmingen), and the anti-mouse VE-cadherin antibody was diluted to 200-fold in blocking solution. The secondary antibody was Alexa Fluor 488 Goat Anti-rat IgG (Life technologies), and the antibody was diluted to 400-fold in blocking solution. The prepared specimens were observed and photographed under a confocal laser microscope (Leica). Z-stack images were acquired using a confocal laser microscope and analyzed for the expression level (fluorescence intensity) of VE-cadherin.

### (2) Results

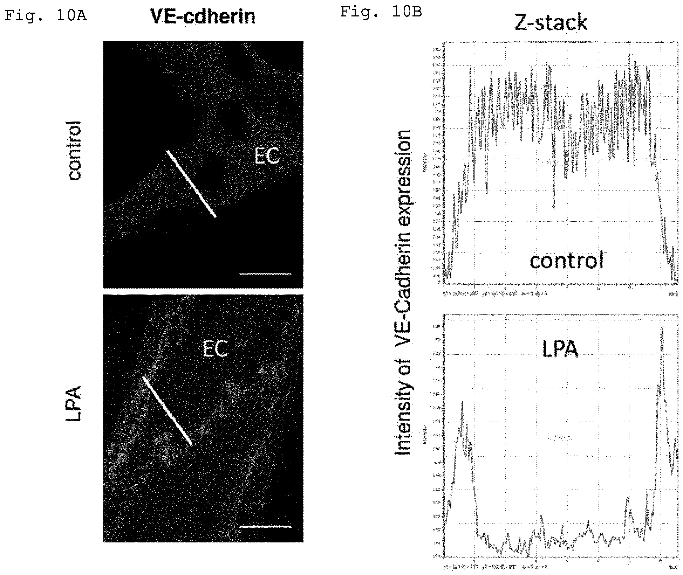

The results are shown in Figs. 10A and 10B. Fig. 10A is the confocal laser microscopic images of the specimens. VE-cadherin was stained with green fluorescence, and are shown with white color in the attached figures. Fig. 10B is charts showing the expression level (fluorescence intensity) of VE-cadherin determined from Z-stack images acquired along the direction indicated by the inclined white bar in Fig. 10A. The lower panel in Fig. 10A indicated that VE-cadherin localized on the cell-cell junctions in the LPA group. In the control group, VE-cadherin was weakly expressed in the cells. The charts from the Z-stack images in Fig. 10B also shows a strong fluorescence intensity on the cell membrane in the LPA administration group, indicating that a large amount of the VE-cadherin molecules localized on the cell membrane. The results revealed that LPA induces the localization of the endothelial adherens junction protein VE-cadherin to the cell membrane, thereby inducing cell-cell adhesion of vascular endothelial cells.

### Example 8: Induction of cell-cell adhesion of vascular endothelial cells by LPA derivative

As described in Example 7, LPA was revealed to strengthen the cell-cell adhesion of endothelial cells, thereby inducing the improvement of network formation of vascular endothelial cells and the improvement of vascular permeability. Based on the results, to examine whether the strengthening of the cell-cell adhesion of endothelial cells can also be achieved by the LPA derivative VPC 31144 (S) (N-{(1S)-2-hydroxy-1-[(phosphonooxy)methyl]ethyl}(9Z)octade c-9-enamide), the cell-cell adhesion of endothelial cells was analyzed by a method involving monitoring electric signals.

### (1) Experimental method

In vitro barrier function of the cell-cell junctions of vascular endothelial cells in the presence of LPA or VPC 31144 (S) was analyzed using a real-time cell ECIS-Zθ analyzer (Applied Biophysics).

LPA and VPC 31144 (S) were purchased from Avanti Polar Lipids, Inc. VPC 31144 (S) is an LPA derivative that exhibits an agonist effect on LPAR4. LPA and VPC 31144 (S) were each dissolved in 50% ethanol as a 10 mM LPA stock solution and stored at -30°C. Before use, the LPA stock solution was thawed and homogenized with an ultrasonic cleaner (SND Co., Ltd.) for 1 minute. The solution was diluted in a serum-free culture medium supplemented with 0.1% BSA to a concentration of 10 µM.

Mouse pancreatic vascular endothelial cell line (MS-1) was used. The cells were suspended in DMEM (Sigma) containing 5% FBS. The cell suspension was seeded on a well plate with electrodes (8W10E+, 8 wells) specially designed for the analyzer at a density of 1 × 10⁵ cells in 400 µL per well, and the cells were cultured overnight in an incubator to confluence. The medium was replaced by serum-free culture medium containing 0.1% BSA, and the cells were incubated for 4 hours. The medium was replaced by culture medium (serum-free culture medium containing 0.1% BSA) containing 10 µM LPA or VPC 31144 (S). The medium of the control cultures was replaced by serum-free culture medium containing 0.1% BSA. After replacement of the medium, resistance between cells (Rb) was measured in real time to evaluate the barrier function.

### (2) Results

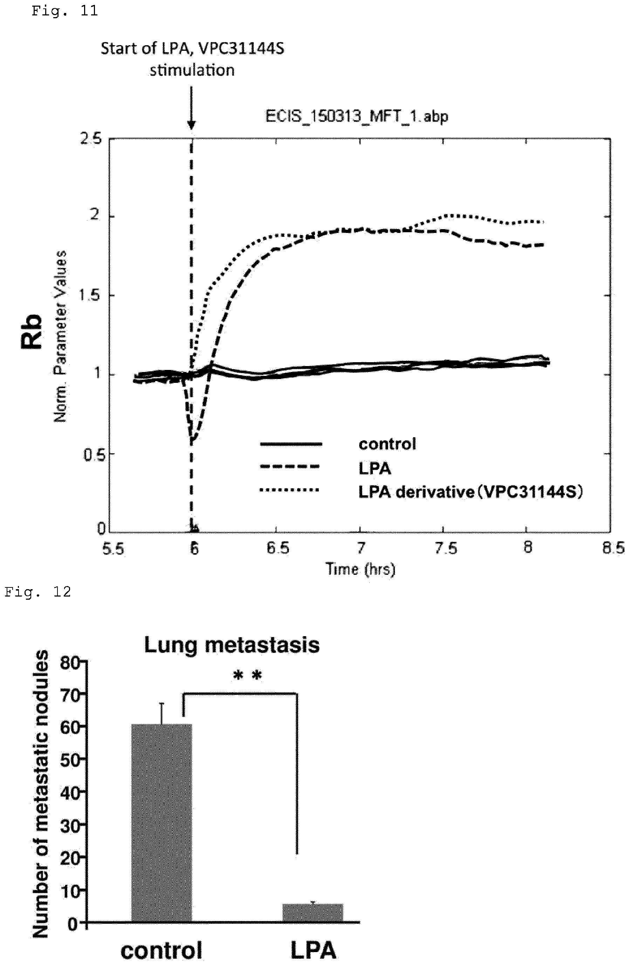

The results are shown in Fig. 11. In Fig. 11, the X axis represents the time elapsed, and the Y axis represents the electric resistance (Rb). A higher Rb value indicates a stronger cell-cell adhesion. Addition of LPA increased the electric signals as compared with the control, and the increased barrier function in endothelial cells was maintained over a long period of time. Similarly, addition of VPC 31144 also increased the barrier function in endothelial cells. Addition of VPC 31144 more rapidly increased the barrier function immediately after the administration, as compared with addition of LPA. The results revealed that not only LPA but also an LPA derivative that exhibits an agonist effect on LPAR4 is useful for the improvement of vascular permeability and the normalization of the vascular network in diseases involving abnormal blood vessel formation.

### Example 9 : Study of inhibition of cancer metastasis from primary lesion by LPA administration

Hypoxia in tumor tissues is previously described to potentially cause malignant conversion of the cancer cells that facilitates metastasis and invasion of the cancer cells. Improvement of the blood flow in tumors may prevent the reduction in oxygen partial pressure in tumors and inhibit malignant conversion of cancers. Also as described above, maintenance of the cell-cell adhesion in the endothelial cells may prevent the migration and invasion of cancer cells to blood vessels, may resulting in inhibition of metastasis. Based on these assumptions, the effects of LPA on cancer metastasis was investigated using a melanoma cell line with high metastatic ability (B16-BL6 cells).

### (1) Experimental method

B16-BL6 cells (1 × 10⁶ cells in 100 µL PBS per animal) were subcutaneously injected into C57BL/6 NCrSlc mice aged 8 weeks (females, SLC, Inc.). On day 7 post-inoculation of B16-BL6 cells, mice with a tumor volume of 30 to 50 mm³ were selected. The mice were divided into two groups: control group and LPA group (n = 3). LPA (3 mg/kg in 100 µL PBS) or PBS (100 µL) was administered intraperitoneally. LPA or PBS was administered once daily until day 21 post-inoculation. On day 42 post-inoculation of B16-BL6 cells, mice were euthanized and the lung was harvested. The number of metastatic colonies in the harvested lung was counted under a stereoscopic microscope (Leica). For the statistical analysis, Student's t-test was performed.

### (2) Results

The results are shown in Fig. 12. As is apparent from Fig. 12, lung metastasis was significantly inhibited in the LPA group as compared with the control group (p < 0.01). The results confirmed that LPA-induced improvement in the tumor blood flow and in vascular endothelial cell-cell adhesion inhibits the malignant conversion and metastasis of cancer cells.

### Example 10: Expression analysis of LPA receptors in vascular endothelial cells of tumor tissues

### (1) Experimental method

LLC cells were subcutaneously inoculated into C57BL/6 NCrSlc mice aged 8 weeks (females, SLC, Inc.) to develop tumors in the same manner as in Example 1. The tumors were harvested, and vascular endothelial cells with CD45 (blood marker) negative and CD31 (vascular endothelial cell marker) positive were collected with FACS Aria (BD Biosciences). From the collected vascular endothelial cells (CD45⁻CD31⁺), a total RNA was extracted with RNAeasy kit (Qiagen). A cDNA was synthesized from the total RNA using ExScript RT reagent Kit (Takara Bio Inc.). The mRNA expression levels of LPA receptors (LPARs) 1-6 were analyzed by real-time PCR using the cDNA. As a control, the mRNA expression level of the glycolytic enzyme GAPDH (glyceraldehyde-3-phosphate dehydrogenase) was measured. For real-time PCR, Stratagene Mx300P (Stratagene) was used.

The primers used in the real-time PCR were as follows.
LPAR1
   5'-CCGCTTCCATTTCCCTATTT-3' (SEQ ID NO: 1)
   5'-AAAACCGTGATGTGCCTCTC-3' (SEQ ID NO: 2)
LPAR2
   5'-CCATCAAAGGCTGGTTCCT-3' (SEQ ID NO: 3)
   5'-TCCAAGTCACAGAGGCAGTG-3' (SEQ ID NO: 4)
LPAR3
   5'-TTCCACTTTCCCTTCTACTACCTG-3' (SEQ ID NO: 5)
   5'-TCCACAGCAATAACCAGCAA-3' (SEQ ID NO: 6)
LPAR4
   5'-GCCCTCTCTGATTTGCTTTT-3' (SEQ ID NO: 7)
   5'-TCCTCCTGGTCCTGATGGTA-3' (SEQ ID NO: 8)
LPAR5
   5'-AGCGATGAACTGTGGAAGG-3' (SEQ ID NO: 9)
   5'-GCAGGAAGATGATGAGATTGG-3' (SEQ ID NO: 10)
LPAR6
   5'-TGTGCCCTACAACATCAACC-3' (SEQ ID NO: 11)
   5'-TCACTTCTTCTAACCGACCAG-3' (SEQ ID NO: 12)
GAPDH
   5'-AACTTTGGCATTGTGGAAGG-3' (SEQ ID NO: 13)
   5'-GGATGCAGGGATGATGTTCT-3' (SEQ ID NO: 14)

### (2) Results

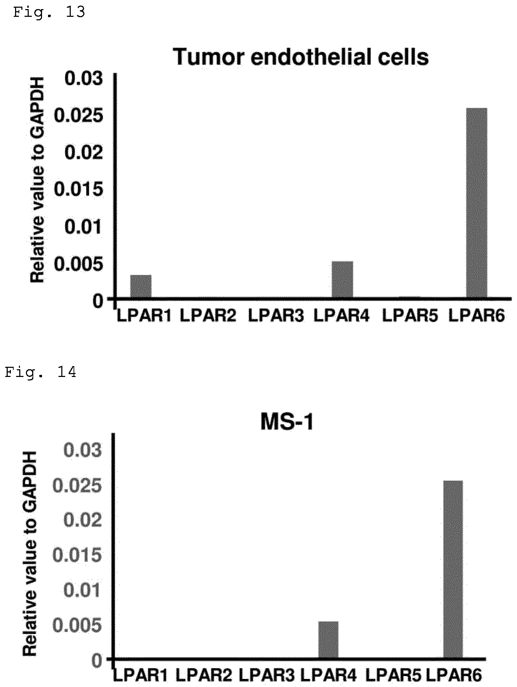

The results are shown in Fig. 13. The expression levels of the LPA receptors are expressed as a relative value to the expression level of GAPDH. As is apparent from Fig. 13, LPAR1, LPAR4 and LPAR6 were expressed in vascular endothelial cells of tumor tissues, and the expression of LPAR2, LPAR3 and LPAR5 were under the detection limit.

### Example 11: Expression analysis of LPA receptors and function analysis of LPAR4 in vascular endothelial cell line

### (1) Expression analysis of LPA receptors

A total RNA was extracted from MS-1 cells (mouse pancreatic vascular endothelial cell line) and the mRNA expression levels of LPA receptors (LPARs) 1-6 were analyzed by real-time PCR in the same manner as in Example 9.

The results are shown in Fig. 14. The expression levels of the LPA receptors are expressed as a relative value to the expression level of GAPDH. As is apparent from Fig. 14, LPAR4 and LPAR6 were expressed in MS-1 cells, and the expression levels of LPAR1, LPAR2, LPAR3 and LPAR5 were under the detection limit.

### (2) Function analysis of LPAR4

LPAR4-specific siRNA (siRNA ID: s95367, Life Technologies) was introduced into MS-1 cells using Lipofectamine 2000 (Invitrogen) to knock down LPAR4. As control cells, MS-1 cells having the introduced control siRNA were used. The control cells and the LPAR4 knockdown cells were each seeded on a glass bottom dish (Iwaki) coated with type I collagen at a density of 2.5 × 10⁵ cells in 2 mL of culture medium. The cells were cultured for 3 days until confluence. After washing the cells three times with PBS each for 1 minute, a few drops of blocking solution (5% normal goat serum, 1% BSA and 2% skim milk in PBS) was added to the dish, and blocking was performed at room temperature for 30 minutes. Purified Goat Anti-mouse VE-cadherin (BD pharmingen) was added as a primary antibody and incubated at 4°C overnight. After washing the cells three times with PBS (PBST) containing Triton X-100 each for 10 minutes, Alexa Fluor 488 Goat Anti-rat IgG (Molecular Probes) was added as a secondary antibody and incubated at room temperature under light-shielding for 1 hour. All the subsequent procedures were carried out at room temperature under light-shielding. After washing the cells three times with PBST each for 10 minutes, nuclear staining was performed with TO-PRO-3 (life Technologies). The cells were washed twice with PBS each for 10 minutes. Several drops of Vectashield (Vector Laboratories Inc.) were applied to the cells and the cells were covered with glass coverslips. The cells were observed and photographed under a confocal laser microscope.

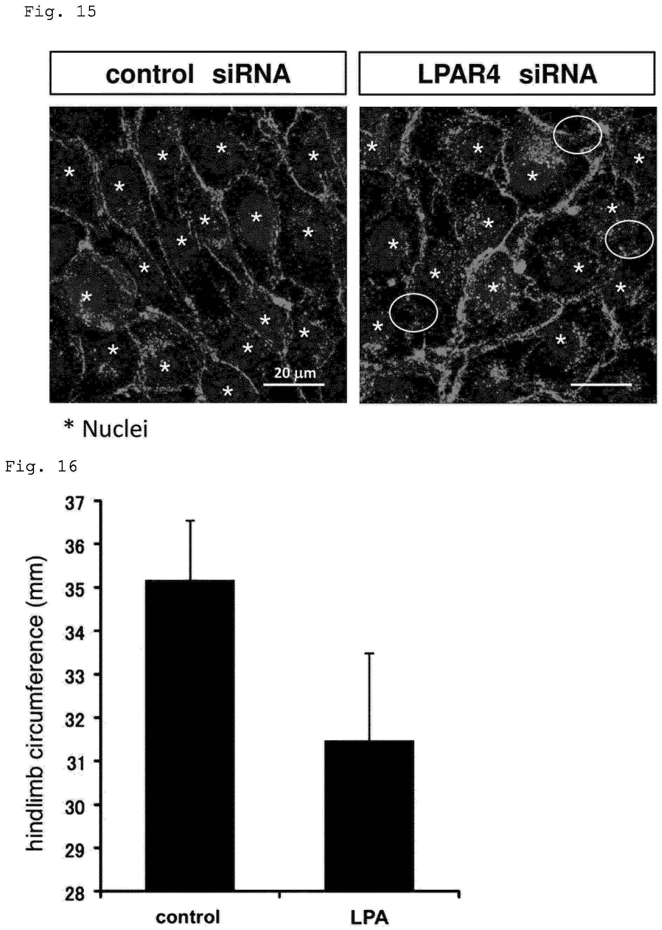

The results are shown in Fig. 15. The control cells cultured until confluence (left) showed a cobble stone-like arrangement and the cells were connected to the neighboring cells. The LPAR4 knockdown cells (right) showed no difference in the number of proliferated cells, but the cell-cell junctions were disrupted. The control cells were spindle shaped, but the LPAR4 knockdown cells did not show such a shape and had a swelled shape. Loss of VE-cadherin-mediated cell-cell junctions occurred in many of the LPAR4 knockdown cells (encircled in white in the right panel of Fig. 15), indicating the gaps between the cells.

### Example 12: Effects of LPA on diseases involving blood vessel formation with increased vascular permeability

Deterioration of pathological conditions due to increased vascular permeability of newly formed blood vessels is observed not only in tumors but also in other diseases, such as inflammatory diseases, diabetic retinopathy, age-related macular degeneration. Based on this, analyses were performed to examine whether the effect of LPA for inhibiting an increase in the vascular permeability of newly formed blood vessels is exhibited not only in tumor blood vessels but also in other disease models.

### 12-1 Hindlimb ischemia model

A hindlimb ischemia model is used as a pathological model of Buerger's disease and chronic arteriosclerosis obliterans. In the production of such a model, a blood vessel in the hindlimb of a mouse is dissected to induce ischemia in the region supplied by the blood vessel. The surgical dissection also causes inflammation. The ischemia and inflammation induce angiogenesis. The newly formed blood vessels have increased permeability, which induces edema in the muscles of the hindlimb. An experiment was performed to determine whether LPA inhibits such increased permeability of newly formed blood vessels induced by inflammation or ischemia.

### (1) Experimental method

C57BL/6 NCrSlc mice aged 6 weeks (females, SLC, Inc.) were used. An incision was made in the inguinal skin of the right hindlimb under anesthesia with pentobarbital (Kyoritsu Seiyaku Corporation). The femoral vein was ligated at the proximal end, and the saphenous artery and vein were ligated at the distal end. The arteries and veins were excised along with their side branches. The skin incision was closed with a sterilized surgical suture (Natsume Seisakusho). The animal was placed in a 37°C incubator until the animal recovered from anesthesia. After the recovery, LPA (3 mg/kg in 100 µL PBS) was intraperitoneally administered to LPA group (n = 3), and PBS (100 µL) was intraperitoneally administered to control group (n = 3). The administration was performed three times in total, once daily for consecutive three days. At 24 hours after the final administration, the hindlimb circumference was measured.

### (2) Results

The results are shown in Fig. 16. The hindlimb circumference in the LPA administration group was significantly reduced as compared with the control group, indicating the inhibition of edema.

### 12-2 Age-related macular degeneration model

Exudative age-related macular degeneration is an ocular disease caused by choroidal neovascularization. The disease remains the leading cause of blindness in the developed nations. The newly formed blood vessels in the choroid are different from healthy blood vessels in that they have increased vascular permeability and are leaky. The leakage of the blood components from the blood vessels causes retinal edema and subretinal fluid accumulation, leading to visual impairments. Drugs that inhibit VEGF, which is involved in the development and growth of new blood vessels in the choroid, have been clinically applied, but a complete cure for the visual impairments has not been discovered yet. Under these circumstances, there has been a demand for the development of a further effective therapy. An experiment was performed to determine whether LPA inhibits such increased permeability of newly formed blood vessels in the choroid.

### (1) Experimental method

C57BL/6 NCrSlc mice aged 8 weeks (females, SLC, Inc.) were used. Tropicamide (Santen Pharmaceutical) was dropped in the eyes of the mice under anesthesia with pentobarbital (Kyoritsu Seiyaku Corporation) to dilate the pupil. The retina surrounding the optic disc of the ocular fundus was irradiated with a laser (Ultima 2000 SE) at four sites in each of the eyes of the mice under observation with a slit lamp microscope for the induction of choroidal neovascularization. The laser irradiation conditions were 150 mW, 0.05 seconds, and 75 µm. LPA (3 mg/kg in 100 µL PBS) was intraperitoneally administered to LPA group (n = 3) on days 5 and 6 after laser irradiation, and PBS (100 µL) was intraperitoneally administered to control group (n = 3) on days 5 and 6 after laser irradiation. On day 7 after laser irradiation (at 48 hours after first administration of LPA), FITC-labeled 70 kDa dextran (Sigma) was administered to the tail vein of the mice under anesthesia with pentobarbital. The dextran was prepared as a solution by dissolving and diluting the FITC-labeled 70 kDa dextran in PBS to a concentration of 5 mg/mL, and 50 µL of the solution was used for the administration. At 10 minutes after dextran administration, the eyeballs were collected. After immersion of the collected eyeballs in 4% paraformaldehyde (PFA) /PBS for 2 hours, the cornea, crystalline lens, and retina were removed from the eyeballs to prepare choroidal flat mounts. The flat mounts were washed with PBS at 4°C. Washing was continued for 6 hours, and PBS was renewed every 30 minutes. The specimens were blocked with blocking solution (5% normal goat serum, 1% BSA and 2% skim milk in PBS) at room temperature for 2 hours. Anti-mouse CD31, Purified Hamster Anti-PECAM-1 (MAB1398Z, Millipore) antibody was used as a primary antibody. The antibody was diluted to 200-fold in blocking solution and a few drops of the antibody were applied to the specimens. The specimens were incubated at 4°C overnight. The specimens were washed six times with PBST each for 30 minutes, and further with PBS for 30 minutes. As a secondary antibody, Alexa Fluor 488 Goat Anti-Hamster IgG (Jackson ImmunoResearch Laboratories) was used. The antibody was diluted to 400-fold in blocking solution and a few drops of the antibody were applied to the specimens. The specimens were incubated under light-shielding for 6 hours. The specimens were washed six times with PBST each for 30 minutes. Several drops of Vectashield were applied to the specimens and the specimens were covered with glass coverslips. The sections were observed and photographed under a confocal laser microscope.

### (2) Results

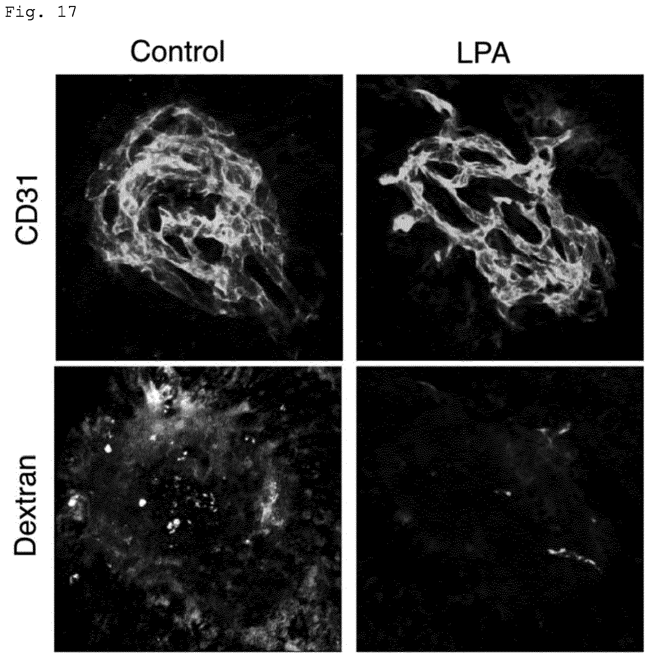

The results are shown in Fig. 17. The upper panels (CD31) are photographs of the tissue specimens containing vascular endothelial cells immunostained with anti-CD31 antibody. Vascular endothelial cells were stained in fluorescent green, and are shown in white color in the attached figures. The lower panels (Dextran) are photographs showing the leakage of the fluorescence-labeled dextran from the newly formed blood vessels. As shown in the upper panels, no significant difference was observed in the size of the newly formed blood vessels in the choroid between the LPA and control groups. However, as shown in the lower panels, the leakage of dextran was significantly inhibited in the LPA group, indicating that LPA has inhibitory effect on an increase in vascular permeability. The results revealed that LPA inhibits an increase in vascular permeability in age-related macular degeneration, thereby ameliorating the pathologies.

The present invention is not limited to each of the embodiments and Examples described above, and various modifications are possible within the scope of the claims. Embodiments obtainable by appropriately combining the technical means disclosed in different embodiments of the present invention are also included in the technical scope of the present invention. The contents of the scientific literature and the patent literature cited herein are hereby incorporated by reference in their entirety.

## Claims

1. A drug delivery enhancer comprising, as an active ingredient, a substance that activates a lysophospholipid receptor, the enhancer being intended to be used in combination with a therapeutic drug for a disease involving abnormal blood vessel formation, thereby enhancing the delivery of the therapeutic drug for a disease involving abnormal blood vessel formation to an affected area.

2. The drug delivery enhancer of claim 1, wherein the substance that activates a lysophospholipid receptor is a lysophospholipid, a precursor thereof or a derivative thereof.

3. The drug delivery enhancer of claim 1 or 2, wherein the disease involving abnormal blood vessel formation is a solid cancer, age-related macular degeneration, rheumatoid arthritis, psoriasis, scleroderma, systemic lupus erythematosus, vasculitis syndrome, vasculo-Behcet's disease, diabetic retinopathy, diabetic nephropathy, arteriosclerosis, chronic arteriosclerosis obliterans, Buerger's disease, pulmonary fibrosis, cerebral infarction, a serious infection or cardiac failure.

4. The drug delivery enhancer of claim 3, wherein the disease involving abnormal blood vessel formation is a solid cancer, age-related macular degeneration, chronic arteriosclerosis obliterans, or Buerger's disease.

5. The drug delivery enhancer of any one of claims 1 to 4, wherein the lysophospholipid is one or more selected from lysophosphatidic acid, lysophosphatidylserine, lysophosphatidylcholine, lysophosphatidylethanolamine, lysophosphatidylinositol, lysophosphatidylglycerol, sphingosine-1-phosphate, sphingosylphosphorylcholine and platelet activating factor (PAF).

6. The drug delivery enhancer of claim 5, wherein the lysophospholipid is lysophosphatidic acid.

7. A pharmaceutical composition for treating a disease involving abnormal blood vessel formation, the composition comprising, as an active ingredient, a substance that activates a lysophospholipid receptor.

8. The pharmaceutical composition of claim 7, wherein the substance that activates a lysophospholipid receptor is a lysophospholipid, a precursor thereof or a derivative thereof.

9. The pharmaceutical composition of claim 8, which has one or more effects selected from inhibitory effect on tumor growth, inhibitory effect on cancer metastasis, enhancing effect on immunity against a tumor, and inhibitory effect on an increase in vascular permeability.

10. The pharmaceutical composition of claim 8 or 9, which is used in combination with another drug for treating a disease involving abnormal blood vessel formation.

11. The pharmaceutical composition of claim 10, wherein said another drug for treating a disease involving abnormal blood vessel formation is a therapeutic drug for a cancer.

12. The pharmaceutical composition of claim 10 or 11, which enhances the delivery of said another drug used in combination to an affected area of a disease involving abnormal blood vessel formation.

13. The pharmaceutical composition of any one of claims 7 to 12, wherein the disease involving abnormal blood vessel formation is a solid cancer, age-related macular degeneration, rheumatoid arthritis, psoriasis, scleroderma, systemic lupus erythematosus, vasculitis syndrome, vasculo-Behcet's disease, diabetic retinopathy, diabetic nephropathy, arteriosclerosis, chronic arteriosclerosis obliterans, Buerger's disease, pulmonary fibrosis, cerebral infarction, a serious infection or cardiac failure.

14. The pharmaceutical composition of claim 13, wherein the disease involving abnormal blood vessel formation is a solid cancer, age-related macular degeneration, chronic arteriosclerosis obliterans, or Buerger's disease.

15. The pharmaceutical composition of any one of claims 7 to 14, wherein the lysophospholipid is one or more selected from lysophosphatidic acid, lysophosphatidylserine, lysophosphatidylcholine, lysophosphatidylethanolamine, lysophosphatidylinositol, lysophosphatidylglycerol, sphingosine-1-phosphate, sphingosylphosphorylcholine and platelet activating factor (PAF).

16. The pharmaceutical composition of claim 15, wherein the lysophospholipid is lysophosphatidic acid.

17. A method for screening for a therapeutic drug for a disease involving abnormal blood vessel formation, the method comprising selecting a substance that activates a lysophospholipid receptor specifically expressed in vascular endothelial cells of an affected area.
